(19) Europäisches Patentamt
European Patent Office
Office européen des brevets

(11) EP 2 319 864 A2

(12) EUROPEAN PATENT APPLICATION

(43) Veröffentlichungstag:
**11.05.2011 Patentblatt 2011/19**

(21) Anmeldenummer: **10185265.5**

(22) Anmeldetag: **21.03.2001**

(51) Int Cl.:
***C07K 14/435*** *(2006.01)* ***A01K 67/033*** *(2006.01)*
***C12N 15/12*** *(2006.01)* ***A61K 49/00*** *(2006.01)*

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **22.03.2000 DE 10014109**

(62) Dokumentnummer(n) der früheren Anmeldung(en) nach Art. 76 EPÜ:
**01933732.8 / 1 268 535**

(71) Anmelder: **Sanofi-Aventis Deutschland GmbH**
**65929 Frankfurt (DE)**

(72) Erfinder:
- **Höner, Marius**
  **81377 München (DE)**
- **Link, Wolfgang**
  **81475 München (DE)**
- **Cassata, Giuseppe**
  **81373 München (DE)**
- **Tovar, Karl-Heinz**
  **61352 Bad Homburg (DE)**
- **Baumeister, Ralf**
  **79104 Freiburg (DE)**

(74) Vertreter: **Körner, Kathrin**
**Sanofi-Aventis Deutschland GmbH**
**Patente Deutschland**
**Industriepark Höchst**
**Gebäude K801**
**65926 Frankfurt am Main (DE)**

Bemerkungen:
This application was filed on 01-10-2010 as a divisional application to the application mentioned under INID code 62.

# (54) Nematodes as model organisms for the investigation of neurodegenerative diseases, in particular parkinson's disease, use and method

(57) Die vorliegende Erfindung betrifft Nematoden als Modellorganismen zur Untersuchung neurodegenerativer Erkrankungen und insbesondere der Parkinsonschen Erkrankung, Verwendungen und Verfahren zum Auffinden von Substanzen und Genen, die bei der Behandlung solcher Erkrankungen eingesetzt werden können, sowie die Identifizierung eines Nematodengens aus C. elegans, das zu dem mit der Parkinsonschen Erkrankung assoziierten menschlichen Parkingen homolog ist.

Die vorliegende Erfindung betrifft insbesondere solche Nematoden, die eine aberrante oder fehlende Expression mindestens eines Gens, das mit der Parkinsonschen Erkrankung in Zusammenhang steht, bevorzugt eines Parkingens und/oder eines α-Synucleingens aufweisen. Die erfindungsgemäßen Organismen können insbesondere zur Identifizierung und Charakterisierung von Arzneimitteln zur Behandlung derartiger Erkrankungen eingesetzt werden.

EP 2 319 864 A2

SEQUENZPROTOKOLL

<110> Sanofi-Aventis Deutschland GmbH

<120> Nematoden als Modellorganismen zur Untersuchung neurodegenerativer Erkrankungen und insbesondere der Parkinsonschen Erkrankung, Verwendungen und Verfahren zum Auffinden von Substanzen und Genen, ...

<130> DEAV2000/A068 EPT1

<140>
<141>

<150> 100 14 109.9
<151> 2000-03-22

<160> 12

<170> PatentIn Ver. 2.1

<210> 1
<211> 1161
<212> DNA
<213> Caenorhabditis elegans

<220>
<221> CDS
<222> (1)..(1158)
<223> Nukleinsäuresequenz der Parkin-cDNA

<400> 1
atg tct gat gaa atc tct ata tta ata caa gat aga aaa aca ggt caa 48
Met Ser Asp Glu Ile Ser Ile Leu Ile Gln Asp Arg Lys Thr Gly Gln
1 5 10 15

cgt agg aat cta aca ctt aat ata aat ata act gga aat atc gaa gat 96
Arg Arg Asn Leu Thr Leu Asn Ile Asn Ile Thr Gly Asn Ile Glu Asp
20 25 30

ctc aca aaa gat gtg gaa aag ctc acc gaa att ccc agc gat gag ctg 144
Leu Thr Lys Asp Val Glu Lys Leu Thr Glu Ile Pro Ser Asp Glu Leu
35 40 45

gaa gtg gtt ttc tgt ggg aaa aag tta tca aaa tca acg att atg agg 192
Glu Val Val Phe Cys Gly Lys Lys Leu Ser Lys Ser Thr Ile Met Arg
50 55 60

gat ttg tca ctg aca cct gca aca caa atc atg ctt ctc cgt cca aag 240
Asp Leu Ser Leu Thr Pro Ala Thr Gln Ile Met Leu Leu Arg Pro Lys
65 70 75 80

ttc aat agt cac aac gaa aac ggt gct act act gca aaa ata aca aca 288
Phe Asn Ser His Asn Glu Asn Gly Ala Thr Thr Ala Lys Ile Thr Thr
85 90 95

gat tct tca att ctc gga agc ttc tac gtg tgg tgc aaa aat tgt gac 336
Asp Ser Ser Ile Leu Gly Ser Phe Tyr Val Trp Cys Lys Asn Cys Asp
100 105 110

gac gtc aag cgc ggc aaa ctg cgg gtt tat tgc caa aaa tgc tcg tca 384
Asp Val Lys Arg Gly Lys Leu Arg Val Tyr Cys Gln Lys Cys Ser Ser
115 120 125

acc tct gtt cta gtc aaa tct gaa ccc cag aac tgg tcc gac gtt ctc 432
Thr Ser Val Leu Val Lys Ser Glu Pro Gln Asn Trp Ser Asp Val Leu
130 135 140

aaa agc aag aga ata ccg gcg gtc tgc gaa gaa tgc tgt act cca ggt 480

```
Lys Ser Lys Arg Ile Pro Ala Val Cys Glu Glu Cys Cys Thr Pro Gly
145                 150                 155                 160

ctt ttc gct gaa ttc aag ttc aaa tgt cta gcc tgc aac gat ccg gcc      528
Leu Phe Ala Glu Phe Lys Phe Lys Cys Leu Ala Cys Asn Asp Pro Ala
                165                 170                 175

gca gct cta act cac gta cgc gga aat tgg caa atg acc gag tgc tgt      576
Ala Ala Leu Thr His Val Arg Gly Asn Trp Gln Met Thr Glu Cys Cys
            180                 185                 190

gtt tgt gat ggg aag gag aaa gtg atc ttc gac ctc gga tgc aat cat      624
Val Cys Asp Gly Lys Glu Lys Val Ile Phe Asp Leu Gly Cys Asn His
        195                 200                 205

att aca tgc caa ttc tgt ttc aga gat tat ttg cta agt caa ctg gaa      672
Ile Thr Cys Gln Phe Cys Phe Arg Asp Tyr Leu Leu Ser Gln Leu Glu
    210                 215                 220

cga ttc ggt ttt gtc aat cag ccg ccg cat ggc ttc acc att ttc tgc      720
Arg Phe Gly Phe Val Asn Gln Pro Pro His Gly Phe Thr Ile Phe Cys
225                 230                 235                 240

ccc tat cca ggg tgc aat aga gtg gta caa gat gtg cac cat ttc cac      768
Pro Tyr Pro Gly Cys Asn Arg Val Val Gln Asp Val His His Phe His
                245                 250                 255

att atg ggt cag acg tcg tac agc gaa tac caa cgg aaa gcc acc gag      816
Ile Met Gly Gln Thr Ser Tyr Ser Glu Tyr Gln Arg Lys Ala Thr Glu
            260                 265                 270

cga ttg att gcc gtg gac gac aag ggt gtg act tgc ccg aat gtc tcg      864
Arg Leu Ile Ala Val Asp Asp Lys Gly Val Thr Cys Pro Asn Val Ser
        275                 280                 285

tgt ggg cag agc ttc ttc tgg gag ccc tat gat gac gat gga aga tcc      912
Cys Gly Gln Ser Phe Phe Trp Glu Pro Tyr Asp Asp Asp Gly Arg Ser
    290                 295                 300

cag tgt cca gat tgt ttt ttt tcg ttt tgc aga aag tgc ttc gaa aga      960
Gln Cys Pro Asp Cys Phe Phe Ser Phe Cys Arg Lys Cys Phe Glu Arg
305                 310                 315                 320

aat tgt gtg tgc cag agc gaa gac gat ctc acc cga act aca att gac      1008
Asn Cys Val Cys Gln Ser Glu Asp Asp Leu Thr Arg Thr Thr Ile Asp
                325                 330                 335

gcg act aca aga aga tgc cca aaa tgc cac gtg gca acc gaa cgg aac      1056
Ala Thr Thr Arg Arg Cys Pro Lys Cys His Val Ala Thr Glu Arg Asn
            340                 345                 350

ggc gga tgt gct cac att cac tgt acc tcg tgt gga atg gat tgg tgt      1104
Gly Gly Cys Ala His Ile His Cys Thr Ser Cys Gly Met Asp Trp Cys
        355                 360                 365

ttc aag tgc aag aca gaa tgg aag gaa gag tgt caa tgg gac cat tgg      1152
Phe Lys Cys Lys Thr Glu Trp Lys Glu Glu Cys Gln Trp Asp His Trp
    370                 375                 380

ttt aat taa                                                          1161
Phe Asn
385


<210> 2
<211> 386
<212> PRT
<213> Caenorhabditis elegans
```

```
<400> 2
Met Ser Asp Glu Ile Ser Ile Leu Ile Gln Asp Arg Lys Thr Gly Gln
1               5                   10                  15

Arg Arg Asn Leu Thr Leu Asn Ile Asn Ile Thr Gly Asn Ile Glu Asp
            20                  25                  30

Leu Thr Lys Asp Val Glu Lys Leu Thr Glu Ile Pro Ser Asp Glu Leu
        35                  40                  45

Glu Val Val Phe Cys Gly Lys Lys Leu Ser Lys Ser Thr Ile Met Arg
    50                  55                  60

Asp Leu Ser Leu Thr Pro Ala Thr Gln Ile Met Leu Leu Arg Pro Lys
65                  70                  75                  80

Phe Asn Ser His Asn Glu Asn Gly Ala Thr Thr Ala Lys Ile Thr Thr
                85                  90                  95

Asp Ser Ser Ile Leu Gly Ser Phe Tyr Val Trp Cys Lys Asn Cys Asp
            100                 105                 110

Asp Val Lys Arg Gly Lys Leu Arg Val Tyr Cys Gln Lys Cys Ser Ser
        115                 120                 125

Thr Ser Val Leu Val Lys Ser Glu Pro Gln Asn Trp Ser Asp Val Leu
    130                 135                 140

Lys Ser Lys Arg Ile Pro Ala Val Cys Glu Glu Cys Cys Thr Pro Gly
145                 150                 155                 160

Leu Phe Ala Glu Phe Lys Phe Lys Cys Leu Ala Cys Asn Asp Pro Ala
                165                 170                 175

Ala Ala Leu Thr His Val Arg Gly Asn Trp Gln Met Thr Glu Cys Cys
            180                 185                 190

Val Cys Asp Gly Lys Glu Lys Val Ile Phe Asp Leu Gly Cys Asn His
        195                 200                 205

Ile Thr Cys Gln Phe Cys Phe Arg Asp Tyr Leu Leu Ser Gln Leu Glu
    210                 215                 220

Arg Phe Gly Phe Val Asn Gln Pro Pro His Gly Phe Thr Ile Phe Cys
225                 230                 235                 240

Pro Tyr Pro Gly Cys Asn Arg Val Val Gln Asp Val His His Phe His
                245                 250                 255

Ile Met Gly Gln Thr Ser Tyr Ser Glu Tyr Gln Arg Lys Ala Thr Glu
            260                 265                 270

Arg Leu Ile Ala Val Asp Asp Lys Gly Val Thr Cys Pro Asn Val Ser
        275                 280                 285

Cys Gly Gln Ser Phe Phe Trp Glu Pro Tyr Asp Asp Asp Gly Arg Ser
    290                 295                 300

Gln Cys Pro Asp Cys Phe Phe Ser Phe Cys Arg Lys Cys Phe Glu Arg
305                 310                 315                 320

Asn Cys Val Cys Gln Ser Glu Asp Asp Leu Thr Arg Thr Thr Ile Asp
                325                 330                 335

Ala Thr Thr Arg Arg Cys Pro Lys Cys His Val Ala Thr Glu Arg Asn
            340                 345                 350

Gly Gly Cys Ala His Ile His Cys Thr Ser Cys Gly Met Asp Trp Cys
        355                 360                 365
```

```
Phe Lys Cys Lys Thr Glu Trp Lys Glu Glu Cys Gln Trp Asp His Trp
    370                 375                 380

Phe Asn
385


<210> 3
<211> 6451
<212> DNA
<213> Künstliche Sequenz

<220>
<223> Beschreibung der künstlichen Sequenz: Plasmid
      pLG0126

<400> 3
cgatatcgaa cattgccagc ttcttgcgag cttttctgaa ataatagtgt tttctaaaca 60
cgtaaacatg aaatatttca cttacgcaag tgcttcttcg gcctctctca tatctgcatt 120
cagccgcttt ttggattcct cggaatcttt ccacagcttg cggagacgct caatctcatc 180
aatcaaatga aacattcctg aaacacgtca ttaaaatttg aaaatgataa ttgaaactaa 240
cctatatcct taatatcgaa ttgcggtcgc tgtgagttta gaatcatgtt gaaaatgtga 300
cgcgagtttt cgccgcacac cttctctttt gatgtactgg acttcattct aaaatgtgga 360
aatgattaga aaacgagaaa ctcgcccgaa aataagagaa aaatgcgtga aaaccgtttc 420
aaatttcgtg gaaaacagtt cgaatttgaa gctcgctgcg tttgtctcac acgcgacgcg 480
acccgctacg cttgccatag ggcgcacatg actgcgagga ctagtgtgca caaaaacatg 540
gggcttcaag gcctcgacta gtttttttgaa tttaatgttt aaaactgcaa gcaggcccgc 600
tagcaggaaa tttttttgtt aatttctaag tcaaattttc agctctcatg aagcatgtct 660
gatgaaatct ctatattaat acaagataga aaaacaggtc aacgtaggaa tctaacactt 720
aatgtagtgg acatttcaaa ctttgaatat atacattatt tttttcagat aaatataact 780
ggaaatatcg aagatctcac aaaagatgtg gaaaagctca ccgaaattcc cagcgatgag 840
ctggaagtgg ttttctgtgg gaaaaagtta tcaaaatcaa cgattatgag ggatttgtca 900
ctgacacctg caacgtaggt caagtaaata tttacttata taaataactg gaattgttat 960
ttatataaat aactggaatt gttattcaaa taatattatt tcagacaaat catgcttctc 1020
cgtccaaagt tcaatagtca caacgaaaac ggtgctacta ctgcaaaaat aacaacagat 1080
tcttcaattc tcggaagctt ctacgtgtgg tgcaaaaatt gtgacgacgt caagcgcggc 1140
aaactgcggg tttattgcca aaaatgctcg tcaacctctg ttctagtcaa atctgaaccc 1200
cagaactggt ccgacgttct caaaagcaag agaataccgg cggtctgcga agaatgctgt 1260
actccaggtc ttttcgctga attcaagttc aaatgtctag cctgcaacga tccggccgca 1320
gctctaactc acgtacgcgg aaattggcaa atgaccgagt gctgtgtttg tgatgggaag 1380
gagaaagtga tcttcgacct cggatgcaat catattacat gccaattctg tttcagagtg 1440
agtaagaatc taaatttttt gttgaaattg tttaatttta aaggattatt tgctaagtca 1500
actggaacga ttcggttttg tcaatcagcc gccgcatggc ttcaccattt tctgccccta 1560
tccagggtgc aatagttcgt tcgattttat caaaaccatt caattttctg cagtagtgat 1620
cctgaaaact aattgataga aacaaaaaat cttccaaaaa atacaaatat gttatgtttc 1680
cattttgcaa gtctggcatg gttttttttt tgcaaaaaaa acccccaccc gttctattta 1740
aatttatttt gaaaattttc tcacatgttt caatagtttt tcaatgccga gaaaattgaa 1800
aaaaaaagtt ttaaagaaat taaacagaac atttaattga aaaataactt caggagtggt 1860
acaagatgtg caccatttcc acattatggg tcagacgtcg tacagcgaat accaacggaa 1920
agccaccgag cgattgattg ccgtggacga caagggtgtg acttgcccga atgtctcgtg 1980
tgggcagagc ttcttctggg agccctatga tgacgatgga agatcccagt gtccagattg 2040
ttttttttcg ttttgcaggt attttgagct tctaaatcgg aaattttatc gcaataaata 2100
tcatcgttca gaaagtgctt cgaaagaaat tgtgtgtgcc agagcgaaga cgatctcacc 2160
cgaactacaa ttgacgcgac tacaaggtga tctcagcgat tatccactac aaaaaactgt 2220
aaattcttcc agaagatgcc caaaatgcca cgtggcaacc gaacggaacg gcggatgtgc 2280
tcacattcac tgtacctcgt gtggaatgga ttggtgtttc aagtgcaaga cagaatggaa 2340
ggaagagtgt caatgggacc attggtttaa taggtcgacg gtaccgcggg cccgggatcc 2400
accggtcgcc accatggtga gcaagggcga ggagctgttc accggggtgg tgcccatcct 2460
ggtcgagctg gacggcgacg taaacggcca caagttcagc gtgtccggcg agggcgaggg 2520
cgatgccacc tacggcaagc tgaccctgaa gttcatctgc accaccggca agctgcccgt 2580
gccctggccc accctcgtga ccaccctgac ctacggcgtg cagtgcttca gccgctaccc 2640
cgaccacatg aagcagcacg acttcttcaa gtccgccatg cccgaaggct acgtccagga 2700
gcgcaccatc ttcttcaagg acgacggcaa ctacaagacc cgcgccgagg tgaagttcga 2760
gggcgacacc ctggtgaacc gcatcgagct gaagggcatc gacttcaagg aggacggcaa 2820
catcctgggg cacaagctgg agtacaacta caacagccac aacgtctata tcatggccga 2880
caagcagaag aacggcatca aggtgaactt caagatccgc cacaacatcg aggacggcag 2940
cgtgcagctc gccgaccact accagcagaa cacccccatc ggcgacggcc ccgtgctgct 3000
```

```
gcccgacaac cactacctga gcacccagtc cgccctgagc aaagacccca acgagaagcg 3060
cgatcacatg gtcctgctgg agttcgtgac cgccgccggg atcactctcg gcatggacga 3120
gctgtacaag taaagcggcc gccaccgcgg tggagctccg catcggccgc tgtcatcaga 3180
tcgccatctc gcgcccgtgc ctctgacttc taagtccaat tactcttcaa catccctaca 3240
tgctctttct ccctgtgctc ccacccccta tttttgttat tatcaaaaaa acttcttctt 3300
aatttctttg tttttttagct tcttttaagt cacctctaac aatgaaattg tgtagattca 3360
aaaatagaat taattcgtaa taaaaagtcg aaaaaaattg tgctccctcc ccccattaat 3420
aataattcta tcccaaaatc tacacaatgt tctgtgtaca cttcttatgt ttttttttact 3480
tctgataaat ttttttttgaa acatcataga aaaaaccgca cacaaaatac cttatcatat 3540
gttacgtttc agtttatgac cgcaattttt atttcttcgc acgtctgggc ctctcatgac 3600
gtcaaatcat gctcatcgtg aaaaagtttt ggagtatttt tggaattttt caatcaagtg 3660
aaagtttatg aaattaattt tcctgctttt gctttttggg ggtttcccct attgtttgtc 3720
aagagtttcg aggacggcgt tttcttgct aaaatcacaa gtattgatga gcacgatgca 3780
agaaagatcg gaagaaggtt tgggtttgag gctcagtgga aggtgagtag aagttgataa 3840
tttgaaagtg gagtagtgtc tatggggttt ttgccttaaa tgacagaata cattcccaat 3900
ataccaaaca taactgtttc ctactagtcg gccgtacggg ccctttcgtc tcgcgcgttt 3960
cggtgatgac ggtgaaaacc tctgacacat gcagctcccg gagacggtca cagcttgtct 4020
gtaagcggat gccgggagca gacaagcccg tcagggcgcg tcagcgggtg ttggcgggtg 4080
tcggggctgg cttaactatg cggcatcaga gcagattgta ctgagagtgc accatatgcg 4140
gtgtgaaata ccgcacagat gcgtaaggag aaaataccgc atcaggcggc cttaagggcc 4200
tcgtgatacg cctattttta taggttaatg tcatgataat aatggtttct tagacgtcag 4260
gtggcacttt tcggggaaat gtgcgcggaa cccctatttg tttatttttc taaatacatt 4320
caaatatgta tccgctcatg agacaataac cctgataaat gcttcaataa tattgaaaaa 4380
ggaagagtat gagtattcaa catttccgtg tcgcccttat tccctttttt gcggcatttt 4440
gccttcctgt ttttgctcac ccagaaacgc tggtgaaagt aaaagatgct gaagatcagt 4500
tgggtgcacg agtgggttac atcgaactgg atctcaacag cggtaagatc cttgagagtt 4560
ttcgccccga agaacgtttt ccaatgatga gcacttttaa agttctgcta tgtggcgcgg 4620
tattatcccg tattgacgcc gggcaagagc aactcggtcg ccgcatacac tattctcaga 4680
atgacttggt tgagtactca ccagtcacag aaaagcatct tacggatggc atgacagtaa 4740
gagaattatg cagtgctgcc ataaccatga gtgataacac tgcggccaac ttacttctga 4800
caacgatcgg aggaccgaag gagctaaccg cttttttgca caacatgggg gatcatgtaa 4860
ctcgccttga tcgttgggaa ccggagctga atgaagccat accaaacgac gagcgtgaca 4920
ccacgatgcc tgtagcaatg gcaacaacgt tgcgcaaact attaactggc gaactactta 4980
ctctagcttc ccggcaacaa ttaatagact ggatggaggc ggataaagtt gcaggaccac 5040
ttctgcgctc ggcccttccg gctggctggt ttattgctga taaatctgga gccggtgagc 5100
gtgggtctcg cggtatcatt gcagcactgg ggccagatgg taagccctcc cgtatcgtag 5160
ttatctacac gacggggagt caggcaacta tggatgaacg aaatagacag atcgctgaga 5220
taggtgcctc actgattaag cattggtaac tgtcagacca agtttactca tatatacttt 5280
agattgattt aaaacttcat ttttaattta aaaggatcta ggtgaagatc ctttttgata 5340
atctcatgac caaaatccct taacgtgagt tttcgttcca ctgagcgtca gaccccgtag 5400
aaaagatcaa aggatcttct tgagatcctt tttttctgcg cgtaatctgc tgcttgcaaa 5460
caaaaaaacc accgctacca gcggtggttt gtttgccgga tcaagagcta ccaactcttt 5520
ttccgaaggt aactggcttc agcagagcgc agataccaaa tactgtcctt ctagtgtagc 5580
cgtagttagg ccaccacttc aagaactctg tagcaccgcc tacatacctc gctctgctaa 5640
tcctgttacc agtggctgct gccagtggcg ataagtcgtg tcttaccggg ttggactcaa 5700
gacgatagtt accggataag gcgcagcggt cgggctgaac ggggggttcg tgcacacagc 5760
ccagcttgga gcgaacgacc tacaccgaac tgagatacct acagcgtgag cattgagaaa 5820
gcgccacgct tcccgaaggg agaaaggcgg acaggtatcc ggtaagcggc agggtcggaa 5880
caggagagcg cacgagggag cttccagggg gaaacgcctg gtatctttat agtcctgtcg 5940
ggtttcgcca cctctgactt gagcgtcgat ttttgtgatg ctcgtcaggg gggcggagcc 6000
tatggaaaaa cgccagcaac gcggcctttt tacggttcct ggccttttgc tggccttttg 6060
ctcacatgtt ctttcctgcg ttatcccctg attctgtgga taaccgtatt accgcctttg 6120
agtgagctga taccgctcgc cgcagccgaa cgaccgagcg cagcgagtca gtgagcgagg 6180
aagcggaaga gcgcccaata cgcaaaccgc ctctccccgc gcgttggccg attcattaat 6240
gcagctggca cgacaggttt cccgactgga aagcgggcag tgagcgcaac gcaattaatg 6300
tgagttagct cactcattag gcaccccagg ctttacactt tatgcttccg gctcgtatgt 6360
tgtgtggaat tgtgagcgga taacaatttc acacaggaaa cagctatgac catgattacg 6420
ccaagcttgc atgcctgcag gtcgactcta g                                6451
```

<210> 4
<211> 3176
<212> DNA
<213> Künstliche Sequenz

<220>
<223> Beschreibung der künstlichen Sequenz: Plasmid pLG0082

<400> 4
aacctggctt atcgaaatta atacgactca ctatagggag accggcagat ctgatatcat 60
cgatgaattc gagctccacc gcggtggcgg ccgctctaga actagtggat ccaccggttc 120
catggttctc cgtccaaagt tcaatagtca caacgaaaac ggtgctacta ctgcaaaaat 180
aacaacagat tcttcaattc tcggaagctt ctacgtgtgg tgcaaaaatt gtgacgacgt 240
caagcgcggc aaactgcggg tttattgcca aaaatgctcg tcaacctctg ttctagtcaa 300
atctgaaccc cagaactggt ccgacgttct caaaagcaag agaataccgg cggtctgcga 360
agaatgctgt actccaggtc ttttcgctga attcaagttc aaatgtctag cctgcaacga 420
tccggccgca gctctaactc acgtacgcgg aaattggcaa atgaccgagt gctgtgtttg 480
tgatgggaag gagaaagtga tcttcgacct cggatgcaat catattacat gccaattctg 540
ctgcaggaat tcgatatcaa gcttatcgat accgtcgacc tcgagggggg gcccggtacc 600
caattcgccc tatagtgagt cgtattacgc gcgctcactg gccgtcgttt tacaacgtcg 660
tgactgggaa aaccctggcg ttacccaact taatcgcctt gcagcacatc ccccttttcgc 720
cagctggcgt aatagcgaag aggcccgcac cgatcgccct tcccaacagt tgcgcagcct 780
gaatggcgaa tgggacgcgc cctgtagcgg cgcattaagc gcggcgggtg tggtggttac 840
gcgcagcgtg accgctacac ttgccagcgc cctagcgccc gctcctttcg ctttcttccc 900
ttcctttctc gccacgttcg ccggctttcc ccgtcaagct ctaaatcggg ggctcccttt 960
agggttccga tttagtgctt tacggcacct cgaccccaaa aaacttgatt agggtgatgg 1020
ttcacgtagt gggccatcgc cctgatagac ggtttttcgc cctttgacgt tggagtccac 1080
gttctttaat agtggactct tgttccaaac tggaacaaca ctcaacccta tctcggtcta 1140
ttcttttgat ttataaggga ttttgccgat ttcggcctat tggttaaaaa atgagctgat 1200
ttaacaaaaa tttaacgcga attttaacaa aatattaacg cttacaattt aggtggcact 1260
tttcggggaa atgtgcgcgg aacccctatt tgtttatttt tctaaataca ttcaaatatg 1320
tatccgctca tgagacaata accctgataa atgcttcaat aatattgaaa aaggaagagt 1380
atgagtattc aacatttccg tgtcgccctt attccctttt ttgcggcatt ttgccttcct 1440
gtttttgctc acccagaaac gctggtgaaa gtaaaagatg ctgaagatca gttgggtgca 1500
cgagtgggtt acatcgaact ggatctcaac agcggtaaga tccttgagag ttttcgcccc 1560
gaagaacgtt ttccaatgat gagcactttt aaagttctgc tatgtggcgc ggtattatcc 1620
cgtattgacg ccgggcaaga gcaactcggt cgccgcatac actattctca gaatgacttg 1680
gttgagtact caccagtcac agaaaagcat cttacggatg gcatgacagt aagagaatta 1740
tgcagtgctg ccataaccat gagtgataac actgcggcca acttacttct gacaacgatc 1800
ggaggaccga aggagctaac cgcttttttg cacaacatgg gggatcatgt aactcgcctt 1860
gatcgttggg aaccggagct gaatgaagcc ataccaaacg acgagcgtga caccacgatg 1920
cctgtagcaa tggcaacaac gttgcgcaaa ctattaactg gcgaactact tactctagct 1980
tcccggcaac aattaataga ctggatggag gcggataaag ttgcaggacc acttctgcgc 2040
tcggcccttc cggctggctg gtttattgct gataaatctg gagccggtga gcgtgggtct 2100
cgcggtatca ttgcagcact ggggccagat ggtaagccct cccgtatcgt agttatctac 2160
acgacgggga gtcaggcaac tatggatgaa cgaaatagac agatcgctga gataggtgcc 2220
tcactgatta agcattggta actgtcagac caagtttact catatatact ttagattgat 2280
ttaaaacttc atttttaatt taaaaggatc taggtgaaga tcctttttga taatctcatg 2340
accaaaatcc cttaacgtga gttttcgttc cactgagcgt cagaccccgt agaaaagatc 2400
aaaggatctt cttgagatcc tttttttctg cgcgtaatct gctgcttgca aacaaaaaaa 2460
ccaccgctac cagcggtggt ttgtttgccg gatcaagagc taccaactct ttttccgaag 2520
gtaactggct tcagcagagc gcagatacca aatactgtcc ttctagtgta gccgtagtta 2580
ggccaccact tcaagaactc tgtagcaccg cctacatacc tcgctctgct aatcctgtta 2640
ccagtggctg ctgccagtgg cgataagtcg tgtcttaccg ggttggactc aagacgatag 2700
ttaccggata aggcgcagcg gtcgggctga acggggggtt cgtgcacaca gcccagcttg 2760
gagcgaacga cctacaccga actgagatac ctacagcgtg agctatgaga aagcgccacg 2820
cttcccgaag ggagaaaggc ggacaggtat ccggtaagcg gcagggtcgg aacaggagag 2880
cgcacgaggg agcttccagg gggaaacgcc tggtatcttt atagtcctgt cgggtttcgc 2940
cacctctgac ttgagcgtcg atttttgtga tgctcgtcag gggggcggag cctatggaaa 3000
aacgccagca acgcggcctt tttacggttc ctggcctttt gctggccttt tgctcacatg 3060
ttctttcctg cgttatcccc tgattctgtg gataaccgta ttaccgcctt tgagtgagct 3120
gataccgctc gccgcagccg aacgaccgag cgcagcgagt cagtgagcga ggaagc 3176

<210> 5
<211> 39
<212> DNA
<213> Künstliche Sequenz

<220>
<223> Beschreibung der künstlichen Sequenz: Primer RB850

<400> 5
gggccgcggc atgcgaatac aatgacgtaa gcgacgtgg 39

<210> 6

<211> 33
<212> DNA
<213> Künstliche Sequenz

<220>
<223> Beschreibung der künstlichen Sequenz: Primer RB851

<400> 6
cccgtcgact catcagacat gcttcatgag agc 33

<210> 7
<211> 32
<212> DNA
<213> Künstliche Sequenz

<220>
<223> Beschreibung der künstlichen Sequenz: Primer RB853

<400> 7
gggccgcggt tcgaatttga agctcgctgc gt 32

<210> 8
<211> 48
<212> DNA
<213> Künstliche Sequenz

<220>
<223> Beschreibung der künstlichen Sequenz: Primer RB916

<400> 8
cgcccgggag ctcgtcgacc tattaaacca atggtcccat tgacactc 48

<210> 9
<211> 20
<212> DNA
<213> Künstliche Sequenz

<220>
<223> Beschreibung der künstlichen Sequenz: Primer 1-RNA1

<400> 9
cagacaaacc atggttctcc 20

<210> 10
<211> 21
<212> DNA
<213> Künstliche Sequenz

<220>
<223> Beschreibung der künstlichen Sequenz: Primer 1-RNA2

<400> 10
cttactctgc agcagaattg g 21

<210> 11
<211> 30
<212> DNA
<213> Künstliche Sequenz

<220>
<223> Beschreibung der künstlichen Sequenz: Primer RB759

```
<400> 11
cccggctgca gctcaattat tctagtaagc                                   30


<210> 12
<211> 37
<212> DNA
<213> Künstliche Sequenz

<220>
<223> Beschreibung der künstlichen Sequenz: Primer RB110

<400> 12
gtctccatgg atccgaattc tgaaacgttc aaataac                           37
```

**Beschreibung**

Nematoden als Modellorganismen zur Untersuchung neurodegenerativer Erkrankungen und insbesondere der Parkinsonschen Erkrankung, Verwendungen und Verfahren zum Auffinden von Substanzen und Genen, die bei der Behandlung solcher Erkrankungen eingesetzt werden können, sowie Identifizierung eines Nematodengens

Die Erfindung betrifft Nematoden als Modellorganismen zur Untersuchung neurodegenerativer Erkrankungen und insbesondere der Parkinsonschen Erkrankung, die zur Entwicklung von Arzneimitteln zur Behandlung von neurodegenerativen Erkrankungen, einschließlich jenen, bei denen plaqueähnliche Ablagerungen (Amyloidosen) auftreten, und insbesondere zur Behandlung der Parkinsonschen Erkrankung eingesetzt werden können.

Das erfindungsgemäße Tiermodell basiert darauf, daß in einem Nematoden ein Gen, das mit der Entwicklung der Parkinsonschen Erkrankung in Zusammenhang steht, eine aberrante oder fehlende Expression aufweist.

Ein solches Tiermodell kann ebenfalls zur Aufklärung der Stoffwechselwege sowie der Identifizierung von neuen Genen, die an der Parkinsonschen Erkrankung beteiligt sind, dienen.

Hintergrund der Erfindung

Für verschiedene Krankheitstypen stehen bereits transgene Tiermodelle als wertvolle Werkzeuge zur Aufklärung von Erkrankungsprozessen sowie zur Identifizierung und Charakterisierung von präventiv oder therapeutisch wirksamen Arzneimitteln zur Verfügung. Voraussetzung für die Entwicklung solcher Tiermodelle ist insbesondere die Kenntnis von an den jeweiligen Erkrankungsprozessen beteiligten Genen.

Die Parkinsonsche Erkrankung (Morbus Parkinson) ist neben der Alzheimerschen Erkrankung die bekannteste Erkrankung aus der Gruppe von neurodegenerativen Erkrankungen. Sie ist gekennzeichnet durch (1) Verlangsamung aller Bewegungen (Bradykinese), leise und monotone Sprache (Akinese oder Hypokinese), Fehlen der physiologischen Mitbewegungen, gebückte Haltung, kleinschrittiger, z.T. schlurfender Gang, während des Schreibens kleiner werdende Schrift, nicht beeinflussbare Bewegungsstörungen mit Fallneigung nach vorn, zur Seite oder nach hinten, (2) Steifigkeit der Muskulatur (Rigor) und (3) grobschlägiger Ruhetremor (Zittern). Morbus Parkinson ist eine relativ häufig vorkommende Krankheit, die bei ca. 1% der über 60-jährigen, besonders bei Männern auftritt. Die Krankheit wird verursacht durch den Verlust von Dopamin im Striatum, was zur Degeneration von Neuronen in der Substantia nigra führt. Die primäre Ursache, die zu dem Verlust von Dopamin führt, ist nicht bekannt (Dunnett and Björklund, 1999; Olanow and Tatton, 1999). Obwohl die meisten Patienten mit Morbus Parkinson sporadisch auftreten, gibt es eine kleine Gruppe von Patienten, in deren Familien die Erkrankung gehäuft auftritt. Molekulargenetische Analysen in diesen Familien haben zur Identifizierung von mehreren Genen geführt, die, durch Mutationen verändert, ursächlich am Ausbruch der Parkinsonschen Erkrankung beteiligt sind. Eines dieser Gene, welches 1998 durch Nobuyoshi Shimizu, Yoshikuni Mizuno und deren Mitarbeiter entdeckt wurde, wird seither Parkin oder PARK2 genannt (Kitada et al., 1998; Hattori et al., 1998). Es wurde mutiert bei mehreren Familien gefunden, in welchen mehrere Familienmitglieder autosomalen rezessiven juvenilen (vor dem 40. Lebensjahr beginnend) Morbus Parkinson entwickelt haben. Das Protein des Parkin-Gens ist unter anderem charakterisiert durch eine Ubiquitin-Domäne im N-Terminus, zwei RING-Fingerähnliche Motive im C-Terminus und eine IBR (in between ring fingers)-Domäne.

Ein weiteres Gen, welches ebenfalls als ursächlich an der Parkinsonschen Erkrankung beteiligt identifiziert wurde, ist das -Synucleingen (Polymeropoulos et al., 1997). -Synuclein tritt insbesondere in den fibrillären, intrazytoplasmatischen Einschlüssen (Lewy bodies) auf, welche bei der Parkinsonschen Erkrankung auftreten. In den Lewy bodies sind neben dem präsynaptischen Protein α-Synuclein zahlreiche Proteine wie Ubiquitin, und Neurofilament vertreten. Mutationen (A53T und A30P) im α-Synucleingen auf dem menschlichen Chromosom 4q21-q22 führen zu einer autosomal dominanten Form des Morbus Parkinson (Polymeropoulos et al., 1997; Kruger et al., 1998). Es ist noch ungeklärt, wie diese Mutationen Morbus Parkinson auslösen können. Das dominante Vererbungsmuster und die Tatsache, daß α-Synuclein in Lewy bodies in fibrillären Aggregationen vorliegt, deren Bildung durch beide Mutationen beschleunigt werden kann, läßt auf Toxizität als Mechanismus (toxic gain of function) schließen. In einer neueren Studie konnte eine Folge von 12 Aminosäuren in der Mitte des α-Synucleins identifiziert werden, die für die Aggregation in vitro verantwortlich und in dem nicht aggregierenden, sehr ähnlichen Protein ß-Synuclein abwesend ist (Giasson et al., 2000). α-Synuclein wurde bisher nur bei Vertebraten identifiziert, wobei bei allen bisher bekannten Arten Threonin statt wie beim Menschen Alanin in der Position 53 der Aminosäuresequenz zu finden ist (Clayton and George, 1998).

Transgene Tiermodelle, die verschiedene Varianten des humanen α-Synuclein exprimieren, wurden bisher für die Maus und Drosophila etabliert. Die neuronale Expression von α-Synuclein in der Maus führt zu einer fortschreitenden Akkumulation von α-Synuclein in intraneuronalen Einschlüssen, die aber keinen fibrillären Charakter besitzen (Masliah et al., 2000). Demgegenüber zeigen transgene Fliegen, die α-Synuclein panneuronal exprimieren, fibrilläre, den Lewy bodies ähnliche Einschlüsse, den Verlust von dopaminergen Neuronen und eine Beeinträchtigung von Bewegungsfunktionen (Feany and Bender, 2000). Dabei ergaben sich keine wesentlichen Unterschiede zwischen der Expression der einzelnen Formen des α-Synuclein (wt, A53T, A30P).

Zusammenfassung der Erfindung

Gegenüber den bereits veröffentlichten Tiermodellen für Morbus Parkinson besteht der Vorteil eines Nematodenmodells, insbesondere des C. elegans-Modells, vor allem in der Eignung für ein Hochdurchsatzverfahren (HTS) (High-Throughput-Screening), der Möglichkeit einer schnelleren genetischen Analyse durch geringere Generationszeit (2-3 Tage, gegenüber Wochen bei Drosophila) und detaillierterem Wissen über molekulare und funktionelle Eigenschaften des Nervensystems in C. elegans.

Da C. elegans in Mikrotiterplatten gehalten werden kann, können mit diesem Testsystem 10.000 und mehr Substanzen in kurzer Zeit per HTS am lebenden Wurm ausgetestet werden.

Die fortschreitende Sequenzierung verschiedener Genome hat gezeigt, daß für viele Humangene homologe Gene in anderen Organismen existieren. Mehr als zwei Drittel aller bisher bekannten kranheitsassoziierten Gene des Menschen konnten auch im C. elegans-Genom nachgewiesen werden. Eine funktionale Austauschbarkeit dieser Gene wurde experimentell in mehreren Fällen gezeigt. Diese Gemeinsamkeiten zeigen die essentielle Funktion dieser Gene für grundlegende biologische Prozesse an. Sie ermöglichen die Etablierung dieser Modellsysteme zur Untersuchung von humanen Genfunktionen, zur Identifizierung von neuen Zielgenen und zur Entwicklung von Medikamenten.

Die vorliegende Anmeldung beschreibt unter anderem ein bislang nicht bekanntes, aus C. elegans stammendes Parkin kodierendes Gen (Fig. 3; SEQ ID NO:1) sowie dessen Genprodukt (SEQ ID NO:2), das durch Sequenzanalyse und Sequenzvergleich von C. elegans-DNA-Sequenzen mit der bekannten Sequenz des humanen Parkingens mit bioinformatischen Methoden aufgefunden werden konnte.

In Kenntnis dieses neuen Gens und mithilfe weiterer mit der Parkinsonschen Erkrankung bekannterweise in Zusammenhang stehender Gene wie z.B. dem $\alpha$-Synucleingen konnte ein Nematodenmodell entwickelt werden, mit dem neurodegenerative Erkrankungen auf genetischem Wege analysiert werden können, was zur gezielten Entwicklung neuartiger Arzneimittel zur Prävention und/oder Behandlung von derartigen Erkrankungen und insbesondere zur Prävention und/oder Behandlung der Parkinsonschen Erkrankung sowie zur Identifizierung potentieller weiterer, derartige Erkrankungen, und insbesondere die Parkinsonsche Erkrankung auslösender Gene dient.

Im Anschluß an die Identifizierung des Parkingens aus C. elegans wurden im Labor des Anmelders C. elegans-Stämme isoliert (siehe Experimenteller Teil), in denen das C. elegans-Parkingen ganz oder teilweise deletiert ist (sogenannte Parkin-Knock-out-Mutanten). Diese Stämme wiesen im Vergleich zum Wildtyp einen veränderten Phänotyp auf.

Dieser Phänotyp wurde verschiedenen Komplementierungsexperimenten unterzogen. Dabei wurden einerseits ein Teil des Cosmides, welches das C. elegans-Parkingen und dessen Promotor enthält, und andererseits das humane Parkingen in den C. elegans Parkin-Knock-out-Stamm eingesetzt, um den Phänotyp des inaktivierten C. elegans-Parkingens zu kompensieren. Mit diesen Experimenten wurde einerseits belegt, daß es sich beim Phänotyp tatsächlich um die Folgeerscheinung der Inaktivierung des C. elegans-Parkingens handelt und andererseits die funktionelle Austauschbarkeit zwischen C. elegans-Gen und humanem krankheitsassoziierten Gen gegeben ist.

Da beim Menschen Mutationen in und Deletionen von ganz unterschiedlichen Bereichen des Parkingens zu erblichem Morbus Parkinson führen, kann man annehmen, daß es sich bei der Ausprägung der Erkrankung um einen Funktionsverlust des Parkin-Genprodukts handeln muß. Wir haben deshalb die C. elegans Parkin-Knock-out-Stämme als Modellsysteme zum Testen von pharmakologisch wirksamen Substanzen entwickelt. Pharmakologisch wirksame Substanzen sind dabei solche, die den Phänotyp supprimieren, d.h. es können also Substanzen identifiziert werden, die den Phänotyp unterdrücken (indem diese z.B. die Beweglichkeit des Wurmes verbessern).

Nicht immer läßt sich das krankheitsrelevante Genprodukt selbst als therapeutische Zielstruktur für die Medikamentenentwicklung nutzen. Dann bietet es sich in einem in vivo-Modell an, den zugehörigen Stoffwechselweg und die involvierten Genprodukte auf ihre Eignung als Zielstruktur zu untersuchen. Die zuvor erwähnten C. elegans Parkin-knock-out-Wurmstämme eignen sich auch hervorragend zur Identifizierung von neuen Genen, die im Stoffwechselweg von Parkin involviert sind. Die Knock-out-Wurmstämme werden dabei einer Mutagenese unterzogen und Tiere, die keinen veränderten Phänotyp mehr aufweisen, sich also wieder normal bewegen, werden herausselektioniert. Diese werden dann einer genetischen Kartierung unterzogen, um das Gen zu identifizieren, welches mutiert zur Supprimierung des Parkin-knock-out-Phänotyps führt. Dieses Zielgen ist dann unter Umständen wieder für eine Medikamentenentwicklung geeignet.

Neben dem auf dem Parkingen basierenden Modell wurde ebenfalls ein transgenes Nematodenmodell entwickelt, bei dem ein humanes $\alpha$-Synucleingen in C. elegans unter der Kontrolle spezifischer C. elegans-Promotoren exprimiert wird. Auch dieses Modell reproduziert wichtige Merkmale der Parkinsonschen Erkrankung und kann somit ebenfalls als Werkzeug zur Entwicklung neuartiger Pharmaka zur Behandlung dieser Erkrankung verwendet werden.

Die Expression des humanen -Synucleingens bzw. von Derivaten des humanen - Synucleingens, insbesondere von Mutanten, die im Menschen erbliche Formen der Parkinsonschen Erkrankung auslösen, wie z.B. der -Synuclein-Mutante A53T mit einem Austausch der Aminosäure Alanin an Position 53 zu Threonin, in C. elegans-Tieren führte z.B. zu einer morphologischen Veränderung im Kopf (anteriores Drittel des Körpers)- und/oder Schwanzbereich bzw. in/an der Vulva (posteriores Drittel des Körpers) der Tiere, die durch eine Schwellung (Zunahme des Durchmessers der Tiere) aufgrund von Funktionsunfähigkeit oder -reduzierung der Aktivität von Muskeln und Neuronen gekennzeichnet ist. Durch die Expression von -Synuclein bzw. seinen Derivaten in C. elegans wird vermutlich die Funktion von Genen, insbesondere solchen, die in Muskeln und Neuronen exprimiert werden, modifiziert.

Gegenstand der Erfindung ist somit unter einem ersten Aspekt ein Nematode, der eine aberrante oder fehlende Expression eines mit der Parkinsonschen Erkrankung in Zusammenhang stehenden Gens, insbesondere eines Parkin- und/oder - Synucleingens aufweist. Dieser Nematode kann als Modellorganismus für neurodegenerative Erkrankungen, einschließlich jenen, bei denen plaqueähnliche Ablagerungen im Nervensystem (Amyloidosen) auftreten, und insbesondere für die Parkinsonsche Erkrankung verwendet werden.

Die aberrante oder fehlende Expression kann dabei beispielsweise das aus C. elegans stammende Parkingen, das humane Parkingen oder irgendein homologes Parkingen aus einem anderen Organismus oder ein -Synucleingen aus einem beliebigen Organismus wie z.B. ein humanes -Synucleingen betreffen.

Insbesondere kann die aberrante oder fehlende Expression des Parkingens durch eine vollständige oder teilweise Deletion dieses Gens oder durch eine zeitweilige Inaktivierung dieses Gens z.B. durch die im Stand der Technik bekannte RNA interference (RNAi)-Technik verursacht werden.

Unter einem besonderen Aspekt weist der Nematode der Erfindung einen mit der aberranten oder fehlenden Genexpression des Parkin- und/oder -Synucleingens assoziierten Phänotyp auf.

Der Phänotyp, der mit der aberranten oder fehlenden Genexpression des Parkingens assoziiert ist, kann beispielsweise ein Chemotaxisdefekt, ein Eiablagedefekt, eine verlangsamte Entwicklungsdauer, eine verringerte Anzahl an Nachkommen, Koordinationsprobleme, ein Defäkationsdefekt, eine verlangsamte Fortbewegung oder eine verkürzte Körperlänge sein.

Der Phänotyp, der mit der aberranten oder fehlenden Genexpression des - Synucleingens assoziiert ist, kann beispielsweise ein Eiablagedefekt, ein Defekt bei der Vulvaformation, Ablagerungen von -Synuclein, eine verlangsamte Entwicklungsdauer oder eine verringerte Anzahl an Nachkommen sein.

Die verschiedenen Phänotypen werden im experimentellen Teil noch näher beschrieben.

Des weiteren umfaßt die Erfindung die Verwendung eines Nematoden der Erfindung als Modellorganismus, insbesondere

- zur Untersuchung der Mechanismen der Krankheitsentstehung, des Krankheitsverlaufs und/oder der Propagation von neurodegenerativen Erkrankungen, einschließlich jenen, bei denen plaqueähnliche Ablagerungen (Amyloidosen) im Nervensystem auftreten, und insbesondere der Parkinsonschen Erkrankung,
- zur Identifizierung und/oder Charakterisierung von Arzneimitteln und Genen zur Prävention oder Behandlung von neurodegenerativen Erkrankungen, einschließlich jenen, bei denen plaqueähnliche Ablagerungen (Amyloidosen) im Nervensystem auftreten, und insbesondere der Parkinsonschen Erkrankung sowie
- zur Identifizierung und/oder Charakterisierung von Wirkstoffen und Genen, die in der Lage sind, die Wirkung von Arzneimitteln, die zur Prävention oder Behandlung von neurodegenerativen Erkrankungen, einschließlich jenen, bei denen plaqueähnliche Ablagerungen (Amyloidosen) im Nervensystem auftreten, und insbesondere der Parkinsonschen Erkrankung eingesetzt werden, zu modifizieren.

Unter weiteren Aspekten umfaßt die Erfindung Verfahren zur Untersuchung einer Substanz oder eines Gens hinsichtlich der Wirksamkeit bei der Behandlung und/oder Prävention von derartigen Erkrankungen sowie Verfahren zur Untersuchung einer Substanz oder eines Gens hinsichtlich einer Eignung, die Wirkung eines Arzneimittels bei der Behandlung und/oder Prävention von derartigen Erkrankungen zu modifizieren, bei welchen die Nematoden der Erfindung Verwendung finden.

Außerdem erstreckt sich die Erfindung auch auf die durch die erfindungsgemäßen Verfahren identifizierten Substanzen und Gene, die zur Herstellung von Arzneimitteln zur Prävention und/oder Behandlung von derartigen Erkrankungen, sowie als Leitsubstanz bzw. Leitgen zur Entwicklung von davon abgeleiteten Substanzen bzw. Genen mit Wirksamkeit bei der Behandlung und/oder Prävention von derartigen Erkrankungen oder von Substanzen bzw. Genen mit einer Eignung, die Wirkung eines Arzneimittels bei der Behandlung und/oder Prävention von derartigen Erkrankungen zu modifizieren, verwendet werden können.

Die Entwicklung neuartiger Arzneimittel zur Behandlung der Parkinsonschen Erkrankung oder anderer neurodegenerativer Erkrankungen, einschließlich solcher, die durch die Ablagerung von Proteinaggregaten im Nervensystem gekennzeichnet sind und insbesondere von einer solchen ausgelöst oder verstärkt werden, kann dabei gleichfalls die Untersuchung von Protein-vermittelten Wechselwirkungen oder die Untersuchung von Faktoren, die durch diese Wechselwirkung betroffen werden, umfassen.

Ein Arzneimittel zur Behandlung derartiger Erkrankungen soll dabei insbesondere zum Heilen der Krankheit, zur Linderung einzelner, mehrerer oder aller Symptome der Erkrankung oder zur Verzögerung ihres Verlaufs geeignet sein.

Eine präventive Behandlung kann auf einen vollständigen Schutz vor Ausbruch der Krankheit oder lediglich eine Verzögerung des Krankheitsausbruchs abzielen. Schließlich betrifft die Erfindung ein isoliertes Nukleinsäuremolekül, das für das Parkingen aus C. elegans kodiert, Homologe und Fragmente von diesem, sowie ein Polypeptid oder Protein, das von einem solchen Nukleinsäuremolekül kodiert wird.

Detaillierte Beschreibung der Erfindung

Spezieller beruht die Erfindung auf der Erkenntnis, daß Gene der Parkin-Genfamilie und Gene der -Synuclein-Genfamilie biochemisch oder genetisch mit der Entstehung, Propagierung oder Verstärkung von neurodegenerativen Erkrankungen, einschließlich jenen, bei denen plaqueähnliche Ablagerungen im Nervensystem auftreten (Amyloidosen), d.h. solchen, die durch die Ablagerung von Proteinaggregaten im Nervensytem ausgelöst oder verstärkt werden, und insbesondere der Parkinsonschen Erkrankung in Zusammenhang stehen.

Dementsprechend ist die Erfindung insbesondere auf Nematoden zur Verwendung als Modellorganismen für neurodegenerative Erkrankungen, einschließlich jenen, bei denen plaqueähnliche Ablagerungen im Nervensystem auftreten, gerichtet, die eine aberrante oder fehlende Expression eines Parkingens und/oder eines - Synucleingens aufweisen.

Die hier in Frage kommenden Gene umfassen aber auch homologe Gene aus anderen Organismen. So ergab ein Sequenzvergleich ausgehend von dem C. elegans-Parkingen beispielsweise homologe Gene in den folgenden Organismen:

| | |
|---|---|
| D. melanogaster: | Parkin (27% identisch, 38% ähnlich) |
| H. sapiens: | PARK2 (Parkin) (27% identisch, 36% ähnlich) |
| M. musculus: | Parkin (25% identisch, 36% ähnlich) |

Diese genannten Gene können somit ebenfalls in den Modellorganismus eingeführt werden.

Unter einer aberranten Genexpression versteht man in Zusammenhang mit der Erfindung insbesondere zumindest eine der folgenden:

- eine verstärkte Genexpression, die zur Erzeugung einer gegenüber dem Wildtyp-Gen erhöhten Menge von Transkriptions- und/oder Translationsprodukt des betreffenden Gens führt,
- eine verringerte Genexpression, die zur Erzeugung einer gegenüber dem Wildtyp-Gen verringerten Menge von Transkriptions- und/oder Translationsprodukt des betreffenden Gens führt,
- eine Expression eines mutierten Gens und/oder
- eine Expression eines zu dem Organismus homologen oder heterologen Gens.

Die Begriffe "aberrante oder fehlende Expression" sind in diesem Zusammenhang stets im Verhältnis zu einem genetisch ansonsten entsprechenden, d.h. identischen Organismus, der jedoch bezüglich dieser Expression Wildtyp-Charakter aufweist, zu verstehen. Bei dem zum Vergleich herangezogenen Bezugsorganismus kann es sich somit z.B. um einen in der Natur vorkommenden reinen Wildtyp-Organismus, um einen Organismus aus einer durch herkömmliche Züchtungsverfahren, wie z.B. durch Kreuzung, erhaltenen Linie oder auch um einen bereits gentechnologisch modifizierten, d.h. bezüglich andersartiger Gene transgenen Organismus, der gegebenenfalls zusätzlich Kreuzungen unterworfen worden sein kann, handeln. Entscheidend ist lediglich, daß der Bezugs- oder Ausgangsorganismus bezüglich der hier relevanten Genexpression Wildtyp-Charakter aufweist.

Das im Rahmen der Erfindung aberrant zu exprimierende Gen kann ein zu dem Zielorganismus homologes oder heterologes Gen sein oder von einem zu dem Zielorganismus homologen oder heterologen Gen durch Mutation abgeleitet sein.

Das aberrant zu exprimierende Gen kann in dem Zielorganismus

- vollständig, d.h. mit allen zugehörigen regulativen Bereichen zusätzlich zu dem oder den kodierenden Bereich(en),
- mit nur einem Teil der regulativen Bereiche zusätzlich zu dem oder den kodierenden Bereich(en) oder
- lediglich in Form der kodierenden Bereiche, d.h. in Kombination mit zu dem Gen heterologen regulativen Bereichen,

vorliegen.

Die kodierenden Bereiche können dabei die Gesamtheit der kodierenden Bereiche des relevanten Gens oder Teile von diesen, die aber zumindest die für die in Zusammenhang mit den genannten Erkrankungen essentielle Funktionalität des Gens bzw. Genprodukts sicherstellen, darstellen. Die kodierenden Bereiche können auch in Form eines fusionierten Gens, d.h. fusioniert an ein andersartiges Protein, z.B. ein Markerprotein, kodierende Bereiche, vorliegen.

So kann die aberrante Genexpression im Rahmen der Erfindung die Expression eines nativen, jedoch zu dem Zielorganismus heterologen Gens darstellen oder umfassen.

Eine verstärkte oder verringerte Genexpression wird in der Regel auf Mutation(en) im Promotorbereich und/ oder anderen regulativen Bereichen, z.B. Enhancer-Sequenzen, "matrix/scaffold attachment regions", u.s.w., des betreffenden, zu dem Zielorganismus homologen oder heterologen Gens oder auf eine Kopplung der kodierenden Genbereiche mit einem stärkeren bzw. schwächeren, zu den kodierenden Genbereichen heterologen Promotor zurückzuführen sein. Eine verstärkte Genexpression kann auch durch zusätzlichen geeigneten Einbau von Expressionsverstärkungselementen, wie Enhancer-Sequenzen und "matrix/scaf-fold attachment regions", in die aberrant zu exprimierenden Gene bewirkt werden.

Im Falle der Expression eines mutierten Genprodukts liegt oder liegen - gegebenenfalls zusätzlich zu Mutationen in regulativen Bereichen - eine oder mehrere Mutation(en) im kodierenden Bereich des zu dem Zielorganismus homologen oder heterologen Gens vor, die zu einer Anderung der Aminosäuresequenz des translatierten Genprodukts im Vergleich zu der Aminosäuresequenz des nativen Genprodukts führen.

Ein gleichzeitiges Vorliegen von Mutationen in die Genexpression regulierenden Bereichen und kodierenden Bereichen soll in diesem Zusammenhang selbstverständlich mit umfaßt sein. Auf diese Weise kann beispielsweise eine Überexpression eines mutierten Proteins in einem Zielorganismus realisiert werden.

Die aberrante Genexpression kann dabei je nach Einzelfall gegebenenfalls die Deletion eines oder mehrerer nativer Gene des Zielorganismus mit umfassen. Beispielsweise könnte es bei Expression eines nativen oder mutierten heterologen Gens in dem Zielorganismus in Einzelfällen hilfreich sein, das homologe Gen gleicher Funktion in dem Zielorganismus zu deletieren. Gleiches könnte in Einzelfällen bei der Expression eines mutierten homologen Gens in einem Zielorganismus sinnvoll sein.

Des weiteren kann es gegebenenfalls bevorzugt sein, die aberrante Genexpression gewebespezifisch zu steuern. Hierzu werden die aberrant zu exprimierenden Gene in der Regel an Promotor- sequenzen angefügt, die eine gewebe- oder zellspezifische Expression, beispielsweise im Nervensystem, insbesondere in Neuronen oder speziellen Neuronenzelltypen, erlauben. Es sind jedoch auch gewebespezifisch wirksame Enhancersequenzen bekannt, die bei geeignetem Einbau in ein zu exprimierendes Gen eine gewebespezifische Erhöhung der Expression bewirken können. Derartige gewebe- bzw. zellspezifische Promotoren und Enhancer sind den Fachleuten bekannt (siehe z.B. Baumeister et al., 1996; Way & Chalfie, 1989; und Hobert et al., 1997).

Zusätzlich oder alternativ kann durch Kopplung des relevanten Gens mit einem induzierbaren Promotor eine durch chemische Stimuli oder physikalische Stimuli (z.B. Temperatur) induzierbare Expression des relevanten Gens in einem Zielorganismus realisiert werden. Hier ist auch der Einsatz entwicklungsspezifischer Promotoren denkbar. In beiden Fällen kann somit der Beginn der Expression des relevanten Gens und damit insbesondere das Auftreten von mit dieser Genexpression in Verbindung stehenden Phänotypen auf einen vom Benutzer frei wählbaren (induzierbarer Promotor) bzw. durch die Wahl des (entwicklungsspezifischen) Promotors steuerbaren Zeitpunkt festgelegt werden. Es ist in diesen Fällen dementsprechend möglich, die Entwicklung des Zielorganismus zunächst eine gewisse Zeit unbeeinflußt von der Expression des relevanten Gens ablaufen zu lassen.

Die aberrante Genexpression wird oftmals die Einschleusung des aberrant zu exprimierenden Gens in Form eines Transgens, das wie erläutert, zu dem Zielorganismus homolog oder heterolog sein kann, mittels eines das Transgen umfassenden Konstrukts in Zellen des Zielorganismus umfassen. Im Falle eines zu dem Zielorganismus homologen Transgens liegt das Transgen nach dem Einschleusen in einer andersartigen genetischen Umgebung vor als das entsprechende, natürlicherweise in dem Zielorganismus enthaltene Gen. Dabei kann das Transgen nach der Einschleusung in die Zellen extrachromosomal in diesen letzteren vorliegen und ausgehend von dieser extrachromosomalen Anordnung exprimiert werden. Alternativ wird das Transgen nach dem Einschleusen in die Zelle in das Genom der Zelle integriert und wird als solches exprimiert.

Für die Transformation oder Transfektion von Organismenzellen mit Konstrukten, die die relevanten Transgene enthalten, und allgemeiner für die Herstellung der erfindungsgemäßen Organismen stehen dem Fachmann eine große Vielzahl von insbesondere speziell auf die verwendeten Organismenzellen abgestimmten Standardtechniken zur Verfügung. Protokolle zu diesen Standardtechniken sind in der Literatur leicht aufzufinden, so daß von weiteren Ausführungen in diesem Zusammenhang abgesehen wird.

Beispielsweise kann ein Verfahren zur Erzeugung eines als Modellorganismus eingesetzten erfindungsgemäßen Nematoden umfassen, in einen hermaphroditischen Nematoden ein exprimierbares Konstrukt, das ein gewünschtes Transgen oder eine gewünschte Nukleinsäuresequenz enthält, einzuschleusen. Dies kann beispielsweise durch Mikroinjektion erfolgen. Nach dem Schlüpfen von Nachkommen aus Eiern des Nematoden läßt man diese sich entwickeln, wonach man mindestens einen Nachkommen identifiziert, der das gewünschte Transgen bzw. die gewünschte Nukleinsäure unter der Kontrolle regulatorischer Sequenzen, die eine - gegebenenfalls gewebe- oder zellspezifische - Expression erlauben, enthält. Für diese Identifizierung kann das für die Transfektion verwendete exprimierbare Konstrukt zusätzlich ein Markergen enthalten, das ein leicht nachweisbares Markerprotein, ggf. als Fusionsprotein, kodiert.

Der so identifizierte Nematoden-Nachkomme kann dann, sofern gewünscht oder erforderlich, gegebenenfalls einem weiteren Züchtungsverfahren, z.B. einer Kreuzung mit anderen Nematoden, die beispielsweise weitere interessante Transgene exprimieren, unterworfen werden, um Linien, die diverse gewünschte Eigenschaften zusätzlich zu der vorstehend erläuterten aberranten oder fehlenden Genexpression aufweisen, zu produzieren.

Wird bei einem erfindungsgemäßen Organismus eine fehlende Expression eines nativen Gens angestrebt, so stehen auch hierfür mehrere Möglichkeiten, wie beispielsweise eine zielgerichtete "knock-out"-Mutation, die im experimentellen Teil ausführlicher dargestellt wird, oder der Einsatz einer spezifischen "antisense"- oder "sense"-Strategie sowie einer gezielten Mutagenese, zur Verfügung.

Eine weitere Möglichkeit, ein Gen zu inaktivieren, ist die Anwendung der RNA interference-Technik, welche bei C. elegans vor ca. zwei Jahren etabliert wurde. Dabei wird doppelsträngige RNA des zu analysierenden Gens in den Wurm eingebracht. Diese RNA wird offensichtlich in kleinere Fragmente geschnitten und kann sich daraufhin im gesamten Tier verteilen. Die RNA-Fragmente interagieren in jeder Zelle mit der entsprechenden messenger-RNA, was einen spezifischen Abbau des Transkripts zur Folge hat (Zamore et al., 2000). Dieses Vorgehen führt zu einer loss-of-function-Mutation mit einem Phänotyp, der dem einer Deletion dieses Gens über den Zeitraum einer Generation sehr nahe kommt.

Es gibt zwei verschiedene Möglichkeiten die doppelsträngige RNA in den Wurm zu transferieren. Die erste Möglichkeit besteht in einer Mikroinjektion, wobei man die einzelsträngige RNA durch in-vitro-Transkription herstellt. Anschließend werden sense- und antisense-RNA miteinander hybridisiert und es entsteht die doppelsträngige Form. Diese wird einmalig in den Wurm injiziert und die F1-Nachkommen des Tieres werden analysiert.

Die zweite Möglichkeit besteht in einer Fütterung, wobei die RNA in einem geeignetem Bakterien-Stamm in vivo synthetisiert wird. Durch Wachstum der Würmer auf diesen Futter-Bakterien gelangt die RNA aus dem Intestinaltrakt in die restlichen Zellen, was zu dem oben bereits beschriebenen Abbau der entsprechenden mRNA führt. Der Vorteil dieses Ansatzes ist, daß es zu einer kontinuierlichen Zufuhr von RNA kommt und daher die interferierende Wirkung länger anhält.

In einer besonderen Ausführungsform der Erfindung liegt in einem erfindungsgemäßen Organismus eine aberrante Expression von mehr als einem der vorstehend genannten Gene vor. Darüber hinaus kann der Organismus auch ein Parkingen oder ein dazu homologes Gen als Transgen und/oder ein -Synucleingen oder ein dazu homologes Gen als Transgen exprimieren.

Die erfindungsgemäßen Organismen sind Nematoden, insbesondere Nematoden der Gattung Caenorhabditis, beispielsweise C. elegans, C. vulgaris oder C. briggsae.

Alle im Rahmen dieser Anmeldung definierten transgenen Tiere können als Modellorganismus für neurodegenerative Erkrankungen, bei denen insbesondere die Mitglieder der -Synuclein-Genfamilie und/oder die Gene der Parkin-Genfamilie beteiligt sind, und insbesondere für die gezielte Entwicklung von neuartigen Medikamenten zur Prävention und/oder Behandlung von derartigen Erkrankungen verwendet werden.

Dabei kann mit Arzneimittelkandidatensubstanzen, insbesondere mit kleinen Molekülen, getestet werden, ob der im jeweiligen Einzelfall beobachtete Phänotyp durch die Einwirkung derartiger Substanzen reduziert oder verstärkt wird. Aus diesem Grund eignen sich die transgenen Tiere, insbesondere C. elegans, und beispielsweise jene, die -Synuclein exprimieren, zum Testen neuartiger pharmakologischer Wirkstoffe.

Insbesondere für die Identifizierung und Charakterisierung von Arzneimitteln und Wirkstoffen weist die Verwendung von Nematoden zahlreiche Vorteile auf. Erfindungsgemäße Nematoden zeigen insbesondere Symptome, die in ganz entsprechender Weise bei menschlichen Patienten mit neurodegenerativen Erkrankungen, einschließlich jenen, bei denen plaqueähnliche Ablagerungen im Nervensystem auftreten, und insbesondere solche mit der Parkinsonschen Erkrankung zu beobachten sind. Dabei sind in Nematoden these Symptome leicht und ohne großen Aufwand sowie bereits kurze Zeit nach dem Schlüpfen der Nematoden, z.B. bereits wenige Tage nach dem Schlüpfen, zu beobachten oder zu bestimmen. Darüber hinaus weisen Nematoden im Vergleich zu anderen Organismen kurze Generationszeiten auf und es besteht die Möglichkeit, große Zahlen von Tieren genetisch zu manipulieren und dementsprechend große Zahlen von transgenen Tieren zu erzeugen. Es bestehen keine Probleme bei der Produktion größerer Zahlen von identischer transgener Nachkommenschaft eines erfindungsgemäßen Nematoden, so daß auch für HTS-Verfahren von großen Zahlen von Arzneimittelkandidaten oder von ganzen Substanzbibliotheken ausreichend Organismen bereitgestellt werden können. Derartige Substanzbibliotheken können Bibliotheken synthetisch erzeugter Substanzen oder solche von Naturstoffen darstellen.

Mit Nematoden können derartige Massenscreenings beispielsweise bequem in Mikrotiterplatten ausgeführt werden, wobei für jede Arzneimittel- oder Wirkstoffkandidatenverbindung eine Vertiefung oder im Falle von Parallelproben entsprechend mehrere Vertiefungen zur Verfügung gestellt werden können.

In einem Verfahren zur Untersuchung einer Substanz hinsichtlich Wirksamkeit bei der Behandlung und/oder Prävention von neurodegenerativen Erkrankungen können dementsprechend beispielsweise erfindungsgemäße Organismen der Substanz ausgesetzt und gegebenenfalls auftretende Veränderungen des Phänotyps, der mit der aberranten oder fehlenden Genexpression des mindestens einen mit derartigen Erkrankungen in Verbindung stehenden Gens, wie sie im Vorangehenden erläutert wurden, assoziiert ist, bestimmt werden.

Dabei zeigt eine Abschwächung des beobachteten Phänotyps, eine Aufhebung des beobachteten Phänotyps oder eine Verzögerung der ohne Arzneimittelbehandlung ansonsten mit der Zeit beobachteten Verschlechterung des beobachteten Phänotyps eine Wirksamkeit der untersuchten Substanz als Arzneimittel bei der Behandlung und/oder Prävention von derartigen Erkrankungen an. Eine Verschlechterung des beobachteten Phänotyps über das ohne Arzneimittelbehandlung beobachtete Maß hinaus oder eine Beschleunigung der normalerweise ohne Arzneimittelbehandlung beobachteten Verschlechterung zeigen eine Kontraindikation der untersuchten Substanz bei derartigen Erkrankungen an.

Zur Sicherung der erhaltenen Ergebnisse wird stets die Beobachtung von Kontrollorganismen, die der zu untersuchenden Substanz nicht ausgesetzt sind, sinnvoll sein. Da in den Zielorganismen in der Regel eine Verschlechterung des Phänotyps, d.h. der in Zusammenhang mit der aberranten oder fehlenden Genexpression des mindestens einen mit derartigen Erkrankungen in Verbindung stehenden Gens beobachteten Symptome, mit der Zeit zu beobachten sein wird, wird es bei vergleichenden Untersuchungen (vergleichende Untersuchungen mit verschiedenen Arzneimittelkandidatensubstanzen, vergleichende Untersuchungen mit und ohne Arzneimittelkandidatensubstanz) in der Regel erforderlich sein, einander entsprechende Organismen gleichen Alters, z.B. zum gleichen Zeitpunkt geschlüpfte Nematoden der gleichen Linie, einzusetzen.

In einem Verfahren zur Untersuchung einer Substanz hinsichtlich einer Eignung, die Wirkung eines Arzneimittels bei der Behandlung und/oder Prävention von neurodegenerativen Erkrankungen, einschließlich jenen, bei denen plaqueähnliche Ablagerungen im Nervensystem auftreten, zu modifizieren, können beispielsweise folgende Schritte auszuführen sein:

- einen erfindungsgemäßen Organismus dem Arzneimittel auszusetzen und die aufgrund der Arzneimittelwirkung auftretenden Veränderungen des Phänotyps, der mit der aberranten oder fehlenden Genexpression des mindestens einen mit derartigen Erkrankungen in Verbindung stehenden Gens, wie sie im Vorangehenden erläutert wurden, assoziiert ist, zu bestimmen,
- mindestens ein weiteres Exemplar des Organismus der in dem vorigen Schritt verwendeten Art dem Arzneimittel in Gegenwart der Substanz auszusetzen und gegebenenfalls auftretenden Veränderungen des Phänotyps mit den aufgrund der Arzneimittelwirkung allein auftretenden Veränderungen des Phänotyps, der mit der aberranten oder fehlenden Genexpression des mindestens einen mit derartigen Erkrankungen in Verbindung stehenden Gens, wie sie im Vorangehenden erläutert wurden, assoziiert ist, zu vergleichen.

Die Zugabe der zu untersuchenden Substanz kann vor, gleichzeitig mit oder nach dem Zusatz des jeweils eingesetzten Arzneimittels zu dem den erfindungsgemäßen Organismus enthaltenden Assaysystem erfolgen. Es kann auch eine gemeinsame Vorinkubation von Substanz und Arzneimittel vor dem Zusatz zu dem Assaysystem vorgenommen werden.

Dabei zeigt eine in Gegenwart der Substanz verstärkte Abschwächung des beobachteten Phänotyps, eine in Gegenwart der Substanz neu auftretende Aufhebung des beobachteten Phänotyps oder eine in Gegenwart der Substanz stärkere zeitliche Verzögerung der in Gegenwart von Arzneimittel ohne Zusatz der Substanz ansonsten beobachteten Veränderungen des beobachteten Phänotyps eine Wirksamkeit der untersuchten Substanz als Wirkungsmodifizierungsmittel, das die Arzneimittelwirkung des in dem Assay eingesetzten Arzneimittels bei der Behandlung und/oder Prävention von derartigen Erkrankungen verstärkt, (= Agonistenwirkung) an. Eine in Gegenwart der Substanz beobachtete Verschlechterung des beobachteten Phänotyps im Vergleich zu dem in Gegenwart von Arzneimittel allein beobachteten Phänotyp, eine vollständige Aufhebung der Arzneimittelwirkung oder eine geringere Verzögerung der normalerweise trotz Gegenwart des Arzneimittels beobachteten Verschlechterung zeigen eine Kontraindikation (= Antagonistenwirkung) der untersuchten Substanz bei derartigen Erkrankungen an.

Hinsichtlich der zu Vergleichszwecken einzusetzenden Bezugsorganismen, einschließlich Organismen ohne Behandlung mit Arzneimittel und Substanz sowie Organismen unter ausschließlicher Behandlung mit der Substanz, d.h. ohne Arzneimittelbehandlung, gilt das in den vorangehenden Absätzen Gesagte entsprechend.

Die mittels der erfindungsgemäßen Organismen und Verfahren erfolgende Identifizierung oder Charakterisierung von Arzneimitteln und Modifizierungssubstanzen kann auch ausgehend von Substanzgemischen, z.B. Naturstoffextrakten, Fermentationsbrühen, erfolgen, wobei zunächst das Substanzgemisch getestet und bei nachgewiesener Wirksamkeit eine Trennung oder Fraktionierung des Gemisches in Einzelsubstanzen, gegebenenfalls schrittweise in Richtung immer höherer Reinheit, vorgenommen wird, um schließlich zu dem reinen Wirkstoff oder der reinen Modifizierungssubstanz zu gelangen. Im Verlauf der Trennung oder Fraktionierung wird dementsprechend in der Regel das erfindungsgemaße Verfahren entsprechend häufig wiederholt werden, um die den Wirkstoff oder die Modifizierungssubstanz jeweils enthaltende Fraktion zu identifizieren.

Die vorgelegten Definitionen der erfindungsgemäßen Verfahren sollen somit auch Substanzen umfassen, die in Form eines Substanzgemisches mit anderen Verbindungen vorliegen.

Die Erfindung erstreckt sich gleichfalls auf Substanzen mit Wirksamkeit bei der Behandlung und/oder Prävention von neurodegenerativen Erkrankungen, einschließlich jenen, bei denen plaqueähnliche Ablagerungen im Nervensystem auftreten, und Substanzen mit einer Eignung, die Wirkung eines Arzneimittels bei der Behandlung und/oder Prävention von derartigen Erkrankungen zu modifizieren, die mittels eines der soeben erläuterten erfindungsgemäßen Verfahren identifiziert worden sind. Unter Verwendung dieser Substanzen können verschiedene Arzneimittel hergestellt werden.

Die Erfindung erstreckt sich auch auf die Verwendung einer erfindungsgemäßen Substanz als Leitsubstanz zur Entwicklung von davon abgeleiteten Substanzen mit Wirksamkeit bei der Behandlung und/oder Prävention von neurodegenerativen Erkrankungen, einschließlich jenen, bei denen plaqueähnliche Ablagerungen im Nervensystem auftreten, oder von Substanzen mit einer Eignung, die Wirkung eines Arzneimittels bei der Behandlung und/oder Prävention von derartigen Erkrankungen zu modifizieren.

Die abgeleiteten Substanzen können ausgehend von einer entsprechenden Leitsubstanz durch eine oder mehrere der in diesem Zusammenhang vom Fachman üblicherweise eingesetzten Methoden chemischer Derivatisierung oder Ableitung erhalten worden sein. Nur beispielhaft sei im Falle einer in Form einer organischen Säure vorliegenden Leitsubstanz eine Salzbildung, eine Veresterung, eine Veretherung oder eine Amidbildung genannt oder im Falle einer in Form eines Amins vorliegenden Leitsubstanz eine Salzbildung mit einer organischen oder anorganischen Säure. An der Leitsubstanz vorliegende Hydroxylgruppen können beispielsweise zu Veretherungen oder andersartigen üblichen Umsetzungen genutzt werden. Gleichfalls umfaßt sind jegliche Formen einer Bildung von Additionsverbindungen und Solvaten sowie von Substitutionen, die an dem Grundgerüst einer Leitsubstanz, z.B. an einer darin enthaltenen aromatischen Ringstruktur, vorgenommen werden können. Substitutionen können beispielsweise von Wasserstoffatomen, Halogenatomen, Hydroxylgruppen, Amingruppen, Carbonsäuregruppen oder Alkylgruppen bzw. gegen derartige Gruppen bzw. Atome vorgenommen werden.

Unter einem weiteren Aspekt betrifft die Erfindung ferner die Verwendung der erfindungsgemäßen Organismen

- zur Identifizierung und/oder Charakterisierung von weiteren Genen, die in Zusammenhang mit den Mechanismen der Krankheitsentstehung, des Krankheitsverlaufs und/oder der Propagation von neurodegenerativen Erkrankungen, einschließlich jenen, bei denen plaqueähnliche Ablagerungen im Nervensystem auftreten, insbesondere der Parkinsonschen Erkrankung, stehen;
- zur Identifizierung und/oder Charakterisierung von Genen, die in mutierten oder unmutiertem Zustand zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung wirksam sind sowie
- zur Identifizierung und/oder Charakterisierung von Genen, die in mutierten oder unmutiertem Zustand in der Lage sind, die Wirkung von Arzneimitteln und/oder Genen, die zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung eingesetzt werden, zu modifizieren.

Analog zur Suche nach Arzneimitteln, die einen bestimmten mit neurodegenerativen Erkrankungen, wie der Parkinsonschen Erkrankung, assoziierten Phänotyp unterdrücken bzw. verstärken, können die erfindungsgemäßen Nematoden, z.B. C. elegans, auch dazu benutzt werden, um weitere Gene zu identifizieren, die dieselbe Funktion haben. Dazu wird ein erfindungsgemäßer transgener Stamm oder eine Mutante in einem der vorstehend erläuterten Kandidatengene verwendet. In einem nächsten Schritt wird der Phänotyp, ausgelöst durch das Transgen oder die Mutation, durch zusätzliche Mutation(en) in anderen Genen manipuliert. Dies erfolgt in der Regel durch ungerichtete Mutagenese, beispielsweise durch Einsatz mutagenisierender Chemikalien (z.B. MMS, Methylmethansulfonat; EMS, Ethylmethansulfonat; NNG, N-Methyl-N'-nitro-N-nitrosoguanidin) oder Bestrahlung.

Man kann auf diese Weise zusätzliche Mutationen finden, die den Phänotyp kompensieren oder verstärken (Suppressor- bzw. Enhancer-/Verstärkermutanten). Die betroffenen Gene sind Kandidaten für weitere Gene im selben Signalweg oder Kandidaten für Gene in parallelen Signalwegen. Jedes dieser so neu aufgefundenen Gene stellt daher wieder ein Zielgen für eine pharmakologische Intervention, z.B. für die Identifizierung oder Entwicklung von Arzneimitteln (z.B. mit Wirksamkeit gegenüber einem Genprodukt des fraglichen Gens) oder für die Entwicklung von Gentherapeutika, dar.

Dieser Aspekt stellt neben dem pharmakologischen einen der wichtigsten Aspekte der erfindungsgemäßen Tiermodelle und insbesondere des C. elegans-Tiermodells dar (Stichwort: "pathway dissection", Identifizierung der genetischen Komponenten eines Signalwegs).

Dementsprechend umfaßt die Erfindung auch ein Verfahren zur Identifizierung und/oder Charakterisierung von Genen, die in mutiertem oder unmutiertem Zustand zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung wirksam sind, welches die folgenden Schritte umfaßt,

- an erfindungsgemäßen Organismen eine ungerichtete Mutagenese vorzunehmen,
- an den aus der Mutagenese resultierenden Organismen gegebenenfalls feststellbare Veränderungen des Phänotyps, der mit der aberranten oder fehlenden Genexpression des mindestens einen mit derartigen Erkrankungen in Verbindung stehenden Gens, wie sie im Vorangegangenen erläutert wurden, assoziiert ist, verglichen mit den für die ungerichtete Mutagenese verwendeten Ausgangsorganismen zu bestimmen und

- das oder die Gene, die infolge ihrer Mutation für die in dem vorangegangenen Schritt beobachteten Veränderungen des Phänotyps verantwortlich sind, zu identifizieren.

Die Erfindung betrifft auch ein Verfahren zur Identifizierung und/oder Charakterisierung von Genen, die in mutiertem oder unmutiertem Zustand in der Lage sind, die Wirkung von Arzneimitteln und/oder Genen, die zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung eingesetzt werden, zu modifizieren, welches die Schritte umfaßt:

- an erfindungsgemäßen Organismen eine ungerichtete Mutagenese vorzunehmen,
- aus den aus der Mutagenese resultierenden Organismen Nachkommen identischer Art zu erzeugen,
- an den jeweils identischen Nachkommen einen gegebenenfalls vorliegenden Phänotyp, der mit der aberranten oder fehlenden Genexpression des mindestens einen mit derartigen Erkrankungen in Verbindung stehenden Gens, wie sie im Vorangegangenen erläutert wurden, assoziiert ist, zu bestimmen,
- den in dem vorangegangenen Schritt an den Nachkommen bestimmten Phänotyp mit dem entsprechenden Phänotyp der erfindungsgemäßen Ausgangsorganismen zu vergleichen,
- an den jeweils identischen Nachkommen, die den gleichen Phänotyp wie die Ausgangsorganismen zeigen, Veränderungen des Phänotyps, der mit der aberranten oder fehlenden Genexpression des mindestens einen mit derartigen Erkrankungen in Verbindung stehenden Gens, wie sie im Vorangegangenen erläutert wurden, assoziiert ist, in Gegenwart des zu untersuchenden Arzneimittels oder bei Expression des zu untersuchenden Gens zu bestimmen, und
- die bestimmten Veränderungen des Phänotyps mit den Veränderungen des Phänotyps der Ausgangsorganismen in Gegenwart des zu untersuchenden Arzneimittels oder bei Expression des zu untersuchenden Gens zu vergleichen, um gegebenenfalls auftretende Abweichungen der Phänotypveränderung festzustellen, und
- das oder die Gene, die infolge ihrer Mutation für die in dem vorangegangenen Schritt gegebenenfalls festgestellten Abweichungen der Phänotypveränderungen verantwortlich sind, zu identifizieren.

Die Identifizierung der betreffenden Gene kann auf unterschiedliche, den Fachleuten auf diesem Gebiet bekannte Weisen, beispielsweise durch Rekombinationsanalyse, erfolgen. Kann das gesuchte Gen auf eine begrenzte Zahl von Kandidatengenen eingegrenzt werden, kann beispielsweise auch eine gerichtete Mutation von entsprechenden Ausgangsorganismen durchgeführt werden und die daraus resultierenden Phänotypveränderungen oder Phänotypveränderungsabweichungen bestimmt werden, um zu ermitteln, ob ein Kandidatengen nach Mutation tatsächlich für die Phänotypveränderungen oder Phänotypveränderungsabweichungen in Frage kommt.

In diesem Zusammenhang kann beispielsweise auch eines der folgenden Verfahren zum Einsatz kommen:

(a) ein Verfahren zur Untersuchung eines Gens hinsichtlich Wirksamkeit bei der Behandlung und/oder Prävention von neurodegenerativen Erkrankungen, einschließlich jenen, bei denen plaqueähnliche Ablagerungen im Nervensystem auftreten, welches umfaßt, in einem erfindungsgemäßen Organismus das mutierte oder unmutierte Gen zu exprimieren und Veränderungen des Phänotyps, der mit der aberranten oder fehlenden Genexpression des mindestens einen mit derartigen Erkrankungen in Verbindung stehenden Gens, wie sie im Vorangegangenen erläutert wurden, assoziiert ist, zu bestimmen, oder

(b) ein Verfahren zur Untersuchung einer Gens hinsichtlich einer Eignung, die Wirkung eines Arzneimittels bei der Behandlung und/oder Prävention von neurodegenerativen Erkrankungen, einschließlich jenen, bei denen plaqueähnliche Ablagerungen im Nervensystem auftreten, zu modifizieren, wobei das Verfahren umfaßt,

  - einen erfindungsgemäßen Organismus dem Arzneimittel auszusetzen und die aufgrund der Arzneimittelwirkung auftretenden Veränderungen des Phänotyps, der mit der aberranten oder fehlenden Genexpression des mindestens einen mit derartigen Erkrankungen in Verbindung stehenden Gens, wie sie im Vorangegangenen erläutert wurden, assoziiert ist, zu bestimmen,
  - mindestens ein weiteres Exemplar des Organismus der in dem vorigen Schritt verwendeten Art, in dem zusätzlich das zu untersuchende Gen exprimiert wird, dem Arzneimittel auszusetzen und gegebenenfalls auftretenden Veränderungen des Phänotyps mit den aufgrund der Arzneimittelwirkung allein auftretenden Veränderungen des Phänotyps, der mit der aberranten oder fehlenden Genexpression des mindestens einen mit derartigen Erkrankungen in Verbindung stehenden Gens, wie sie im Vorangegangenen erläutert wurden, assoziiert ist, zu vergleichen.

Für die Identifizierung von Arzneimitteln und Wirksubstanzen können anhand der neu identifizierten Gene entsprechende Organismen mit aberranter oder fehlender Genexpression bezüglich jener neu identifizierten Gene erzeugt werden und Arzneimittelkandidaten ganz entsprechend den vorstehenden Erläuterungen untersucht werden. Alles, was im Vorangegangenen z.B. zu den erfindungsgemäßen Organismen, zur Arzneimittelidentifizierung oder -untersuchung, zur Arzneimittelherstellung usw. gesagt wurde, gilt hier entsprechend.

Schließlich umfaßt die Erfindung auch die Verwendung von dem speziellen, aus C. elegans stammenden Parkingen, zur Herstellung eines Gentherapeutikums zur Prävention oder Behandlung von neurodegenerativen Erkrankungen, einschließlich jenen, bei denen plaqueähnliche Ablagerungen im Nervensystem auftreten, (Amyloidosen) und insbesondere der Parkinsonschen Erkrankung. Derartige Gentherapeutika werden insbesondere dann zum Einsatz kommen, wenn bei einem Patienten oder einer Person, die noch keine Symptome einer derartigen Erkrankung zeigt, Defekte oder Mutationen in einem bestimmten der genannten Gene festgestellt wurden und these Defekte bzw. Mutationen bekanntermaßen mit dem Ausbruch, dem Verlauf oder der Stärke derartiger Erkrankungen und möglicherweise auch der Weitergabe derartiger Erkrankungen an Nachkommen in Verbindung stehen.

Darüber hinaus betreffen weitere Aspekte der Erfindung isolierte Nukleinsäuremoleküle, insbesondere DNA-, aber auch z.B. RNA-Moleküle, die ein Parkin aus C. elegans mit der in Fig. 3 angegebenen Aminosäuresequenz kodieren, insbesondere jene, die eine in Fig. 3 angegebene Nukleinsäuresequenz aufweisen. Von der Erfindung ebenfalls umfaßt werden isolierte Nukleinsäuremoleküle, deren Nukleotidsequenz mindestens 75%, insbesondere mindestens 80%, speziell mindestens 85%, bevorzugt mindestens 90%, besonders bevorzugt mindestens 95% und am meisten bevorzugt mindestens 98% Sequenzähnlichkeit zu den erwähnten Nukleinsäuremolekülen aufweisen, insbesondere solche, die unter stringenten Bedingungen mit den erwähnten Nukleinsäuremolekülen hybridisieren. Vorzugsweise erfolgt eine solche Hybridisierung unter niedrig stringenten Bedingungen, in einer weiteren Ausführungsform auch bei hochstringenten Bedingungen. Im Rahmen dieser Beschreibung wird unter niedrig stringenten Bedingungen eine Hybridisierung in 3 x SSC bei Raumtemperatur bis 65°C und unter hochstringenten Bedingungen eine Hybridisierung in 0,1 x SSC bei 68°C verstanden. SSC steht als Abkürzung für einen 0,15 M Natriumchlorid, 0,015 M Trinatriumcitrat-Puffer.

Die erfindungsgemäßen Nukleinsäuremoleküle mit Nukleinsäuresequenzähnlichkeit zu der in Fig. 3 angegebenen Nukleinsäuresequenz umfassen insbesondere auch allelische Varianten der angegebenen Nukleinsäuresequenz.

Die Erfindung erstreckt sich auch auf die Nukleinsäuremoleküle mit jeweils zu den oben erläuterten Nukleinsäuresequenzen komplementärer Nukleinsäuresequenz.

Der Begriff "isoliertes Nukleinsäuremolekül" bezieht sich im vorliegenden Zusammenhang auf Nukleinsäuremoleküle, die im wesentlichen gereinigt von der Hauptmenge der andersartigen Nukleinsäuremoleküle aus den Ausgangs- bzw. Produzentenzellen vorliegen. Präparationen von erfindungsgemäßen isolierten Nukleinsäuremolekülen können jedoch ohne weiteres weitere Bestandteile, wie Salze, Mediumsubstanzen oder auch restliche Bestandteile der Produzentenzellen, wie z.B. diverse Proteine, enthalten.

Die Erfindung erstreckt sich auch auf Fragmente der vorstehend erläuterten Nukleinsäuremoleküle, insbesondere solche, die Nukleinsäureabschnitte enthalten, die für die Funktion des Expressionsprodukts, d.h. Parkin, erforderliche Aminosäuresequenzbereiche kodieren. Im letzteren Falle kann das Expressionsprodukt eines solchen Nukleinsäurefragments eine dem vollständigen Parkinprotein äquivalente Wirksamkeit/Aktivität oder auch eine erhöhte oder geringere Wirksamkeit/Aktivität aufweisen; bevorzugt ist in jedem Falle, daß das Expressionsprodukt in der Lage ist, die von dem vollständigen Parkinprotein ausgeübte(n) Wirkung(en) in irgendeinem Maße noch auszuüben. Des weiteren erstreckt sich die Erfindung auch auf solche Fragmente, die beispielsweise als hochspezifische Hybridisierungssonden, PCR- oder Sequenzierungsprimer oder auch als "antisense"- oder "sense"-Nukleotide zum spezifischen "homologydependent gene silencing" des Parkingens eingesetzt werden können. Derartige Fragmente werden je nach Verwendungszweck mindestens 15, häufig jedoch 25 und mehr Nukleotide umfassen. Für derartige Verwendungszwecke kann ein erfindungsgemäßes Nukleinsäurefragment auch, sofern erforderlich, ein oder mehrere Markierungs- oder Nachweismolekül(e), z.B. ein Digoxigenin-Molekül oder ein Biotin-Molekül, aufweisen.

Des weiteren umfaßt sind Konstrukte, Vektoren, Plasmide, Cosmide, Bacmide, YACs, BACs, Virus- oder Phagengenome, die eines der erläuterten isolierten Nukleinsäuremoleküle enthalten. Unter Konstrukten werden hier u.a. verstanden:

- Konstrukte, die erfindungsgemäße Nukleinsäuremoleküle bzw. - molekülfragmente unter der Kontrolle eines Promotors enthalten,
- Genkonstrukte, die erfindungsgemäße Nukleinsäuremoleküle bzw. - molekülfragmente fusioniert an andersartige Gene bzw. Genabschnitte enthalten,
- Konstrukte, die z.B. fur eine Verwendung als PCR-Primer erfindungsgemäße Nukleinsäurefragmente ergänzt um zusätzliche Nukleotidsequenzabschnitte, die für eine Klonierung (der aus der PCR-Amplifikation resultierenden Produkte) geeignete Restriktikonsstellen bereitstellen, umfassen.

Ein weiterer Aspekt betrifft die Polypeptide oder Proteine, die von den erläuterten Nukleinsäuremolekülen kodiert werden, insbesondere ein Parkin aus C. elegans mit der in Fig. 3 angegebenen Aminosäuresequenz sowie davon durch Substitution, Modifizierung, Deletion, Insertion oder Hinzufügung von Aminosäuren abgeleitete Polypeptide oder Proteine, die mindestens 75%, insbesondere mindestens 80%, speziell mindestens 85%, bevorzugt mindestens 90%, besonders bevorzugt mindestens 95% und am meisten bevorzugt mindestens 98% Sequenzähnlichkeit zu den erwähnten Aminosäuresequenzen aufweisen. Die Erfindung umfaßt gleichfalls Fragmente der obigen explizit angegebenen Polypeptide oder Proteine und insbesondere solche, die für die Funktion des Parkinproteins aus C. elegans wesentliche Aminosäuresequenzabschnitte enthalten. In einer bevorzugten Ausführungsform der Erfindung sind diese Fragmente in der Lage, die von dem vollständigen Parkinprotein ausgeübte(n) Wirkung(en) in irgendeinem Maße noch auszuüben. Gleichfalls umfaßt sind Fusionsproteine, die erfindungsgemäße Polypeptide, Proteine oder Proteinfragmente fusioniert an ein andersartiges Protein oder einen andersartigen Proteinabschnitt, z.B. ein Marker- oder Indikatorprotein, enthalten.

Die Erfindung erstreckt sich auch auf transgene Organismen, insbesondere Mikroorganismen, z.B. Bakterien, Viren, Protozoen, Pilze und Hefen, Algen, Pflanzen oder Tiere, wie auch Teile, z.B. Zellen, und Vermehrungsmaterial, z.B. Samen, von solchen transgenen Organismen, die eine rekombinante Nukleinsäuresequenz - ggf. in ein Chromosom integriert oder auch extrachromosomal - umfassen, die ein erfindungsgemäßes Nukleinsäuremolekül, wie es soeben erläutert worden ist, als Transgen enthält. Unter einem bevorzugten Aspekt werden die transgenen Organismen das von dem erwähnten Transgen kodierte, Polypeptid oder Protein, d.h. Parkin aus C. elegans oder ein davon abgeleitetes Polypeptid oder Protein, auch exprimieren.

Besonders interessante Verwendungen des Parkingens aus C. elegans bzw. von Mutanten desselben wurden bereits in früheren Abschnitten dieser Beschreibung erläutert.

KURZE ERLAUTERUNG DER FIGUREN

Figur 1: Struktur des C. elegans Parkin-Gens schematisch mit Exons und Introns und der Lokalisation der Deletionen der beiden KO-Mutanten KO1 und K02 (Lage: Parkingen: 308-2027; KO1-Deletion: 804-2550 und KO2-Deletion: 382-1513).

Figur 2: Plasmid pLG0126, welches den C. elegans-Parkin-Promotor und das C. elegans Parkin-GFP-Fusionskonstrukt beinhaltet (SEQ ID NO:3) (Promotor: 2-654; Parkingen: 655-2371; Exon 1: 655-723; Exon 2: 769-914; Exon 3: 1005-1437; Exon 4: 1484-1574; Exon 5: 1854-2058; Exon 6: 2112-2186; Exon 7: 2233-2371; GFP: 2414-3133; 3'-UTR von unc-54: 3179-3923; und Ampicillin-Resistenzgen: 5249-4389).

Figur 3: Aminosäure- und cDNA-Nukleinsäuresequenz des kodierenden Bereichs von Parkin aus C. elegans (SEQ ID NO:1 und 2); Abweichungen der Nukleinsäuresequenz von der im Internet verfügbaren Sequenz (CAB04599.1, Bestimmung nichtexperimentell) sind durch Großbuchstaben gekennzeichnet; Bestimmung durch RNA-Isolation, cDNA-Herstellung und nachfolgende Sequenzierung.

Figur 4: (a) Expressionsmuster von GFP unter der Kontrolle des C. elegans-Parkin-Promotors und (b) Expressionsmuster des C. elegans Parkin-GFP-Fusionskonstrukts (siehe pLG0126 in Fig. 2) unter der Kontrolle von 653 bp des C. elegans-Parkin-Promotors in C. elegans (vgl. experimenteller Teil 2.). Parkin-GFP wird in den allermeisten Neuronen exprimiert. Gezeigt in Fig. 4b sind Neuronen im Kopfbereich (A) und Schwanzbereich (D) mit den dazugehörigen Nomarski-Aufnahmen (B und E). Parkin-GFP wird auch in den allermeisten Muskeln exprimiert. (C) in Fig. 4b zeigt Muskelfasern in der Mitte des Wurms.

Figur 5: Plasmid pLG0082 (SEQ ID NO:4), welches das Exon 3 des C. elegans Parkin-Gens beinhaltet und für die RNAi Versuche verwendet wurde (vgl. experimenteller Teil 12.).

Figur 6: a) Muster der Auftragung auf der Platte beim Chemotaxisassay; b) Ergebnisse des Chemotaxisassays für die C. elegans-Parkin-KO1-Würmer.

Figur 7: Entwicklungsdauer der C. elegans-Wildtypwürmer und der Parkin-KO1-Würmer

Figur 8: Aufnahme von Eiern, die von Parkin-KO2-Würmern gelegt wurden.

Figur 9: Größe der Nachkommenschaft und Anzahl der Eier, die von Parkin-KO1-Würmern gelegt wurden.

Figur 10: Western-Blot mit Extrakten aus Wildtypwürmern (N2) und Würmern, die das in das Genom integrierte, humane α-Synuclein A30P unter der Kontrolle des sel-12 Promotors exprimieren. Die verschiedenen Fraktionen der Proteinextraktion sind unterhalb des Blots gekennzeichnet: HOM, Homogenat; RIPA, RIPA-lösliche Fraktion; S1', erster Waschschritt mit 5% SDS; S1''', dritter Waschschritt mit 5% SDS; UREA, Harnstoff-lösliche Fraktion; FA, Ameisensäure-lösliche Fraktion. Schwarze Pfeile bezeichnen die für α-Synuclein spezifischen Banden.

Figur 11: Analyse trangener Würmer, die α-Synuclein A30P als extrachromosomales Array unter der Kontrolle des Promotors unc-119 exprimieren. Die Immunofärbung (A) wurde mit dem monoklonalen Antikörper 15G7 durchgeführt. (B) Expression des GFP unter der Kontrolle des dat-Promotors. (C) DAPI Färbung; (D) Nomarski-Aufnahme.

Figur 12: Phänotyp transgener Würmern, die das in das Genom integrierte, humane α-Synuclein A30P unter der Kontrolle des sel-12 Promotors exprimieren. (A) Nomarski-Aufnahme eines Wurms dessen Vulva aus dem Körper herausgetreten ist (B; C) Transgener Wurm mit hervortretender Vulva und gestörter Eiablage, in dem sich bereits geschlüpfte Jungtiere befinden.

## EXPERIMENTELLER TEIL

### 1. Bioinformatik

Das C. elegans-Homologe des menschlichen Parkingens wurde durch eine Suche mit dem Programm BLAST auf den C. elegans Sequenzen, die durch das Nationale Zentrum für Biotechnologische Information (www.ncbi.nlm.nih.gov) zur Verfügung gestellt werden, gefunden. Nested PCR Primer-Paare für die Identifizierung von Deletionen wurden mit Hilfe des Programms Primer3 des MIT in Cambridge (www.genome.wi.mit.edu/cgi-bin/primer/primer3_www.cgi) und einem Fenster von 3.2 - 3.3 kb entworfen und synthetisiert.

### 2. Expressionsmuster von Parkin in C. elegans

Die Expression des Parkingens in C. elegans wurde mittels GFP-Reportergenen (GFP = green fluorescent protein) analysiert.

I. In einem ersten Schritt wurde das Plasmid pBY1013 konstruiert, welches 4085 bp des Parkin-Promotors und das GFP-Gen enthielt.
4085 bp aus der 5'-Region des Parkinlocus, amplifiziert als PCR-Produkt mit den Primern

RB850 GGGCCGCGGCATGCGAATACAATGACGTAAGCGACGTGG (SEQ ID NO:5)
RB851 CCCGTCGACTCATCAGACATGCTTCATGAGAGC (SEQ ID NO:6),

wurden als SphI-SalI-PCR-Fragment in den Vektor pPD95.75 (Dr. Andre Fire, Carnegie Institution of Washington, Baltimore, MD, USA) kloniert.
Ein PCR-Produkt aus einer Amplifikation mit den Primern

RB853 GGGCCGCGGTTCGAATTTGAAGCTCGCTGCGT (SEQ ID NO:7)
RB916 CGCCCGGGAGCTCGTCGACCTATTAAACCAATGGTCCCATTGACACTC (SEQ ID NO:8)

wurde in den GFP-Vektor pBY1023 als NheI/SalI-Fragment kloniert. C. elegans-Wildtyptiere wurden mit dem resultierenden Plasmid pBY1 013 transformiert. Semistabile transgene Linien wurden isoliert. Diese zeigten GFP-Expression in zahlreichen Neuronen und Muskeln (siehe Fig. 4a).
Besonders hervorzuheben sind die Expression in

- den "anal sphincter"- und "anal depressor"-Muskeln zur Steuerung der Defäkierung,
- den Vulva- und Uterus-Muskeln zur Steuerung der Eiablage
- im gesamten Pharynx (Muskeln und Neuronen),
- Schwanzneuronen, v.a. solchen mit langem Fortsatz im ventral nerve cord,
- Kopfneuronen im Bereich außerhalb des Pharynx.

II. Herstellung des Plasmids pLG0126 (enthält 653 bp Parkin-Promotor + Parkingen + GFP-Gen als Fusionskonstrukt)
Aus pBY1013 wurde mittels inverser PCR 3432 bp aus der 5'-Promotor-Region des Parkin-Gens herausgeschnitten und mittels Klenow-Polymerase wieder aufgefüllt. In das resultierende Plasmid wurde dann das 1717 bp große Parkingen hinter den Parkin-Promotor und vor das GFP (im Leserahmen mit dem Parkin-Gen) hineinkloniert. Das resultierende Plasmid pLG0126 (Fig. 2) wurde in C. elegans-Wildtyptiere transformiert. Semistabile transgene Linien wurden isoliert. Diese zeigten wie beim Plasmid pBY1013 eine GFP-Expression in zahlreichen Neuronen (z.B. Kopf, Schwanz, Pharynx) und Muskeln (z.B. Pharynx, Vulva, Uterus)(siehe Fig. 4b).

3. Präparation von C. elegans Eiern

Ca. 8.000 jung-adulte Wildtyp-Tiere wurden mit M9-Puffer (22 mM $KH_2PO_4$, 42 mM $Na_2HPO_4$, 86 mM NaCl, 1 mM $MgSO_4$) von den Agarplatten abgespült und in 15 ml Falcon-Röhrchen gesammelt, bis alle Würmer abgelöst waren. Danach wurde für 5 min zentrifugiert und der Überstand abgesaugt. Das Wurm-Pellet wurde in 10 ml M9-Puffer aufgenommen und erneut für 3 min bei 3000 rpm zentrifugiert. Nach Wiederholung letzteren Vorganges wurden 10 ml Bleichlösung (1.44% NaClO, 0.25 M KOH) zum Wurm-Pellet zugegeben. Nach Schütteln für 4 min wurde die Eierpräparation für 30 s bei 1.500 rpm zentrifugiert, der Überstand abgesaugt und das Eier-Pellet in 10 ml M9-Puffer aufgenommen. Danach wurde das Pellet 5 mal mit je 10 ml M9-Puffer gewaschen und jeweils für 1-2 min bei 3.000 rpm zentrifugiert.

4. Herstellung von mutagenisierten Nematoden-Würmern

Für die Herstellung der mutagenisierten Nematoden-Wurmstämme wurde das Eier-Pellet, resultierend aus der Eierpräparation, in 10 ml M9-Puffer aufgenommen und für 20 h bei 20 °C im Überkopfschüttler geschüttelt. Am darauf folgenden Tag wurden die geschlüpften L1-Larven (je 6.000 pro Platte) auf 8 Platten (OP50-Bakterien beimpfte Agarplatten mit 9 cm Durchmesser) ausgesät und für 60-70 h bei 15 °C inkubiert. Nach Abspülen der Tiere mit M9-Puffer und Sammeln in Falcon-Röhrchen wurden sie in M9-Puffer gewaschen bis der Überstand nicht mehr von Bakterien getrübt war (Zentrifugationen waren jeweils für 3 min bei 1.000 rpm). Anschließend wurden 10.000-15.000 Tiere in ein neues Falcon-Röhrchen überführt und mit Trimethylpsoralen (30 μg/ml) versetzt. Die Mischung wurde 15 min auf dem Überkopfschüttler im Dunkeln inkubiert und nach dem Absetzen der Würmer nach 3 min wurden diese auf eine trockene Agarplatte aufgetropft. Nach Eintrocknenlassen der Würmer nach 2-5 min wurden diese für 1 min bei 365 nm mit einer Dosis von 540 $\mu W/cm^2$ bestrahlt. Danach wurden die Würmer mit M9-Puffer abgespült, abzentrifugiert und das Pellet auf 3 neue Platten (9 cm Durchmesser und mit OP50-Bakterien beimpft) gegeben. Nach einer Inkubation für 24 h bei 20 °C im Dunkeln wurde eine Eierpräparation durchgeführt (siehe oben) und über Nacht bei 20 °C inkubiert. Die synchronisierten und mutagenisierten F1/L1-Tiere wurden danach abzentrifugiert und in S-Complete-Medium (200 mM NaCl, 50 mM $KH_2PO_4$, 13 mM Cholesterin, 10 mM Natriumcitrat, 3 mM $CaCl_2$, 3 mM $MgSO_4$, 100 U/l Nystatin, 100-fach PSN-Antibiotik-Mix, Spurenelemente, HB101-Bakterien mit $OD_{600nm}$ von 2.0) aufgenommen (400 Würmer/ml). Die Wurmsuspension wurde in 17-33 Mikrotiterplatten zu je 50 μl pro Well ausgesät und für 5-6 Tage bei 20° C inkubiert. Nach Entnahme von je einem Viertel (12,5 μl pro Well) der Wurmsuspension für die Präparation der genomischen DNA und der Well-Lysate wurden die Wurmplatten zur Langzeitlagerung in Aufbewahrungsboxen gelegt, mit Parafilm verschlossen und bei 15 °C inkubiert.

5. Präparation von gepoolter genomischer DNA

Jeweils ein Viertel der Wurmsuspension (12,5 μl pro Well) einer Originalplatte aus allen 96 Wells wurden in einem 15 ml Falcon-Röhrchen vereinigt und mit 1 ml M9-Puffer versetzt. Die Falcon-Röhrchen wurden zweimal mit je 10 ml M9-Puffer gewaschen (Zentrifugation für 3 min bei 3.000 rpm) und schließlich mit 4 ml Waschpuffer (20 mM Tris-Puffer, pH 7,5, enthaltend 100 mM NaCl und 50 mM EDTA) versetzt. Nach einer Zentrifugation für 3 min bei 3.000 rpm wurde der Überstand bis auf 1 ml abgesaugt und zusammen mit dem Pellet mit einer Pasteurpipette in ein Eppendorf-Röhrchen überführt. In einer Eppendorfzentrifuge wurde das Gemisch für 1 min bei 14.000 rpm abzentrifugiert und der Überstand bis auf 250 μl abgenommen. Dieser Rest wurde mit 350 μl Waschpuffer versetzt und bei -80 °C eingefroren. Nach dem Wiederauftauen wurden 10 μl 10%-iges SDS, 2 μl Proteinase K (20 mg/ml) und 1 μl β-Mercaptoethanol dazugegeben, durch Kippen gemischt und 1 h bei 65 °C verdaut. Ein weiteres Mal wurde 2,5 μl Proteinase K (20 mg/ml) dazugegeben und während 1 h inkubiert. Danach wurde 2,4 μl RNAse A (10 mg/ml) zugegeben, durch Kippen gemischt und während 15 min bei 37 °C inkubiert. Nach Zugabe von 250 μl Proteinfällungslösung (Promega) wurde jedes Eppendorf-Röhrchen 10 s gevortext, 5 min auf Eis gestellt und zentrifugiert (möglichst gekühlt für 10-15 min bei 14.000 rpm). Der Überstand, die DNA enthaltend, wurde vorsichtig abgenommen und in ein neues Eppendorf-Röhrchen gegeben. Zu diesen Überständen wurden 500 μl Isopropanol dazugeben, durch Kippen gemischt, für 5 min auf Eis gestellt und für 10-15 min bei 14.000 rpm zentrifugiert. Der Überstand wurde abgenommen und verworfen. Zum Pellet, die DNA enthaltend, wurde 500 μl 70% Ethanol dazugegeben, durch Kippen gemischt und für 10 min bei 14.000 rpm abzentrifugiert.

Der Überstand wurde ganz sauber abpipettiert. Der Rest des Ethanols wurde wegverdampft, indem die Eppendorf-Röhrchen offen in einen 37 °C warmen Heizblock gestellt wurden. Das übriggebliebene Pellet wurde in 350 μl 10 mM Tris, 1 mM EDTA aufgenommen und über Nacht bei Raumtemperatur stehengelassen. Am nächsten Tag wurde die genomische DNA für 1 h in den 50 °C heißen Heizblock gestellt und auf PCR-Platten verteilt.

6. Herstellung von Well-Lysaten

Von einem Viertel der ausgesäten Wurmsuspension (12,5 μl) wurden Well-Lysate hergestellt, indem 12,5 μl der Wurmsuspension in 12,5 μl Lysepuffer (20 mM Tris-Puffer, pH 8,2 enthaltend 100 mM KCl, 5 mM $MgCl_2$, 0,9% NP-40, 0,9% Tween-20, 0,02% Gelatine, 14,4 μg/ml Proteinase K) aufgenommen, 6 h bei 65 °C verdaut, zur Inaktivierung der Proteinkinase K 15 min bei 95 °C inkubiert und nach kurzem Abkühlen bei -80 °C eingefroren wurde.

7. Identifizierung von Deletionen mittels nested PCR

Genomische DNA-Platten-Pools und Well-Lysate wurden mittels nested PCR auf Deletionen getestet. In der nested PCR wird zuerst eine PCR mit zwei äußeren Primern und dann mit dem daraus entstehenden Produkt eine PCR mit zwei inneren Primern durchgeführt, um eine höhere Spezifität und bessere Amplifikation zu erhalten. Die genomische DNA wurde mit einem Probenstempel (V & P Scientific, San Diego, USA) direkt in ein Gemisch gestempelt (Reaktionsvolumen von 25 μl), welches Taq-Polymerase (Hoffmann-La Roche, 0.5 Units/Reaktion), dNTP-Gemisch (0.2 mM) und Primer (0.4 μM) enthielt. Die Reaktion lief während 35 Zyklen auf 92 °C für 20 Sek., 56 °C für 1 min und 72 °C für variable Zeiten (15-120 s) in Perkin-Elmer Maschinen des Typs 9700.

8. Analyse der Deletionen

PCR-Produkte wurden mittels Agarosegelen (1% Agarose) analysiert und im Falle der Identifizierung von gewünschten Deletionen sequenziert.

9. Isolierung der Mutanten

Die Würmer im identifizierten Well, welches Würmer mit der Deletion im Zielgen, d.h. mit kürzeren PCR-Produkten im Vergleich zu den Wild-Typ Würmern, enthielt, wurden aus der Flüssigkultur herausgenommen und einzeln auf Agar-Platten gegeben. Nach erfolgreicher Selbstvermehrung wurden die Muttertiere mit verschiedenen Primern, die innerhalb und außerhalb der Deletion lagen, auf homozygote und heterozygote Würmern untersucht. Positive Stämme, d.h. Wurmstämme mit Deletionen des Zielgens in entweder einem (heterozygote) oder beiden Allelen (homozygote) wurden fünf Mal mit Wildtyp-Würmern ausgekreuzt, um andere potentielle Mutationen zu beseitigen. Homozygote, vier Mal ausgekreuzte Stämme wurden für die weiteren Experimente verwendet. Hierbei wurden die in Fig. 1 dargestellten Mutanten Parkin KO1 (Stammbezeichnung XY1003) und Parkin K02 (Stammbezeichnung XY1046) erhalten.

10. Phänotypisierung der Parkin-Knock-out-Mutanten KO1 und K02

a) Chemotaxisassay

Methode:

Pro zu testendem Wurmstamm wurden vier Platten aus 1.6% Agar, 5 mM $KH_2PO_4$, 1 mM $CaCl_2$ und 1 mM $MgSO_4$ verwendet. Die Platten wurden wie in Fig. 6a) gezeigt aufgeteilt.
Bei (+) und (-) wurden je 1 μl $NaN_3$ (1 M) aufgetragen. Anschließend wurde bei (+) 1 μl des angemessen verdünnten Lockstoffes und bei (-) 1 μl EtOH (100%) aufgetragen. Die Würmer wurden auf acht großen Agar-Platten mit E. coli kultiviert, mit $H_2O$ und Tween abgewaschen, 1 x bei 1000 rpm gewaschen und in 1 ml $H_2O$ und Tween aufgenommen. Pro Testplatte wurden 10 μl der Wurmsuspension (ca. 100 Würmer) aufgetragen. Durch Senkrechthalten der Testplatte wurden die Würmer entlang einer Linie verteilt, wodurch der identische Abstand zu Lockstoff und Negativkontrolle erzielt wird.
Verwendete Lockstoffe waren Isoamylalkohol (1:200), Diacetyl (1:1000), Pyrazin (10 mg/ml) und 2,4,5-Trimethylthiazol (1:1000). Alle Substanzen wurden in 100% EtOH verdünnt. Die Dauer des Assays betrug 90 min bei RT. Zum Immobilisieren der Würmer wurde anschließend Chloroform in den Deckel der Testplatten gegeben.

Auswertung:

Die Würmer, die sich nach Ablauf der Zeit innerhalb eines Radius von 1.5 cm um den Lockstoff (A) bzw. die Negativkontrolle (B) befanden, wurden ausgezählt. Aus diesen Werten konnte der Chemotaxis-Index wie folgt berechnet werden:

$$\frac{A - B}{A + B}$$

Resultate:

Das Ergebnis ist in Fig. 6b) gezeigt.
Der Parkin KO1-Wurmstamm reagierte nicht auf den Lockstoff Diacetyl (odr). Der Parkin K02-Wurmstamm verhielt sich analog (Ergebnisse nicht gezeigt).

b) Entwicklungsdauer

Methode:

10 Eier des zu testenden Wurmstammes wurden auf je eine kleine Platte gepickt. Ermittelt wurde die Zeit, bis erneut erste Eier auf der Platte liegen.

Resultate:

Beim Wildtyp haben 100% der Würmer nach 72 Stunden wieder erste Eier gelegt.
Beim Parkin KO1-Wurmstamm war dies erst nach über 100 Stunden der Fall. Die Entwicklungsdauer des Parkin KO1 war daher verlangsamt, wie in Fig. 7 gezeigt ist.

c) Analyse frisch gelegter Eier

Methode:

Drei adulte Würmer wurden auf eine frische Platte umgesetzt. Nach 30 bis 60 min konnten die kürzlich gelegten Eier mit Hilfe der Nomarski-Mikroskopie betrachtet werden. Beim Wildtyp-Wurmstamm wurden die Eier während der Gastrulation (28 Zellen), bzw. bis zu einem Zellstadium von 50 Zellen (Beginn der Morphogenese) gelegt. In Fig. 8 sind zwei Eier gezeigt, die von Parkin KO2-Würmern gelegt wurden.

Resultate:

Die vom Parkin K02-Wurmstamm gelegten Eier befinden sich zum Teil in einem zu weit fortgeschrittenen Zellstadium. Parkin K02 hat damit einen partiellen Eiablagedefekt (egl). Dieser wurde ebenfalls bei dem Parkin KO1-Wurmstamm beobachtet.

d) Größe der Nachkommenschaft

Methode:

Pro getestendem Wurmstamm wurden 10 Eier vereinzelt. Sobald diese erneut Eier legten, wurden die Würmer im 24-Stunden-Rhythmus umgesetzt, bis keine Eier mehr gelegt wurden. Die Nachkommen wurden im L1/L2-Stadium ausgezählt. Somit konnte die Gesamt-Nachkommenschaft bestimmt werden. Desweiteren konnte im gleichen Versuch der zeitliche Verlauf der Eiablage skizziert werden.

Resultate:

Das Ergebnis ist in Fig. 9 gezeigt.

e) Bestimmung der Sinuskurve zur Überprüfung der Mobilität

Methode:

5 jung adulte Würmer wurden auf eine frische Platte gesetzt. Die im Bakterienrasen hinterlassene Sinusspur wurde anschließend via Bildschirm hinsichtlich Amplitude und Sinuslänge vermessen. 10 Spuren wurden auf Folie nachgezeichnet, welche dann überlagert wurde.

Resultate:

Die Amplitude des Sinus bei Parkin K01- und KO2-Wurmstämmen entsprach in etwa der Wildtypamplitude. Die Länge des Sinus war jedoch um ca. 20% geringer als bei Wildtyp-Wurmstämmen. Parkin K01- und KO2-Wurmstämme waren folglich bewegungsgestört (unc).

f) Defäkation

Methode:

Gemessen wurde die Zeitspanne zwischen den Ausscheidungen (expulsion), sowie zwischen pBoc (Darmkontraktion kurz vor der Ausscheidung) und der nachfolgenden Ausscheidung. Pro Wurmstamm wurden 10 Tiere betrachtet.

Resultate:

Sowohl der Zyklus expulsion-expulsion als auch der Zyklus
pBoc-expulsion waren bei Parkin KO1 und Parkin KO2 kürzer als bei Wildtyp.

11. Komplementierung des C. elegans Gens durch das homologe Humangen

Für die detaillierte molekulare Charakterisierung der Mutanten wurden diese einem Funktionstest unterzogen. Einerseits wurde getestet, ob die Phänotypen wirklich auf die Deletion im Zielgen zurückzuführen sind und andererseits, ob C. elegans und humanes Zielgen funktionell konserviert sind. Deshalb wurden einerseits das deletierte C. elegans-Zielgen selber und andererseits das homologe Humangen jeweils in die C. elegans knock-out-Stämme mit den Deletionen in den Zielgenen eingesetzt. Beide Gene wurden als cDNA-Konstrukte mit dem C. elegans-Promotor (Fig. 2) eingesetzt, um ein Expressionsmuster zu gewährleisten, welches dem des Zielgens entspricht. Die Konstrukte wurden direkt als Plasmide (50 ng/μl) mit dem Injektionsmikroskop (Zeiss) in den Wurm eingeführt. Die Chemotaxis-, Entwicklungs-, Ei-, Defäkations- und Mobilitätsdefekte konnten sowohl durch die Einführung des C. elegans-Parkin wie auch durch das humane Parkin rückgängig gemacht werden.

12. RNAi-Versuch mit dem C. elegans Parkingen

Zum Zweck der Durchführung eines RNAi-Feeding-Experimentes zur Inaktivierung des Parkin-Gens wurde zunächst das Plasmid pLG0082 (siehe Figur 5) konstruiert: ein 441 bp umfassendes DNA-Fragment, das einen Teil des Exons 3 des Parkin-Gens darstellt, wurde durch PCR-Amplifikation unter Verwendung der Oligonukleotide 1-RNA1 und 1-RNA2 hergestellt. Durch die Wahl der Oligonukleotide wurden eine Ncol (5'seitig)- und eine Pstl (3'seitig)-Restriktionsschnittstelle eingeführ1. Nach der Isolierung des PCR-Fragments wurde es mit den entsprechenden Restriktionsenzymen geschnitten und in den mit den gleichen Enzymen verdauten Vektor pPD129.36 (Dr. Andrew Fire, Carnegie Institution of Washington, Baltimore, MD, USA) ligiert. Auf diese Weise wurde das Parkin-Fragment zwischen zwei Promotoren der T7-RNA-Polymerase positioniert.
Der E. coli-Stamm HT115 (Dr. Lisa Timmons, Carnegie Institution of Washington, Baltimore, MD, USA) wurde mit dem Plasmid pLG0082 transformiert und Ampicillinresistente Kolonien isoliert. Dieser Stamm trägt eine chromosomale Kopie des Gens für die T7-RNA-Polymerase unter der Kontrolle eines lacZ-Promotors und produziert daher nach Induktion mit 1 mM IPTG sense- und antisense-RNA von dem Exon 3 des Parkin-Gens. Das Wachstum der Würmer auf diesen Bakterien führt zu einer Aufnahme der doppelsträngigen Parkin-RNA und einer daraus resultierenden Inaktivierung des Parkin-Gens.
Je Experiment wurden fünf Wildtyp-Würmer im L3-Entwicklungsstadium auf diesen Futterbakterien für etwa 2-3 Tage bei 15°C angezogen. Nach einem Wechsel auf 20°C wurden deren Nachkommen anschließend phänotypisch analysiert.

1-RNA1: CAGACAAACCATGGTTCTCC (SEQ ID NO:9)
1-RNA2: CTTACTCTGCAGCAGAATTGG (SEQ ID NO:10)

Es wurden Ei-, Defäkations- und Mobilitätsdefekte festgestellt.

13. Herstellung einer C. elegans-Mutante, die humanes α-Synuclein exprimiert Klonierung der Konstrukte

Für die Herstellung der Konstrukte, die α-Synuclein unter der Kontrolle des unc-119-Promotors exprimieren, wurden die humane α-Synuclein-cDNA (Dr. Christian Haass, Ludwig-Maximilians-Universität München) und die cDNAs der A53T und A30P Mutanten (Dr. Christian Haass, Ludwig-Maximilians-Universität München) mit Hilfe der PCR mit Primern amplifiziert, die Schnittstellen für SalI enthielten. Die amplifizierten und geschnittenen DNA-Fragmente wurden in die SalI-Schnittstellen des Vektors pPD49.24 (Dr. Andrew Fire, Carnegie Institution of Washington, Baltimore, MD, USA) kloniert, in dem vorher der Promotor des C. elegans-Gens unc-119 eingebracht worden war. Die 5'-Promotor-Region vor dem unc-119-Gen war mittels PCR und zwei Primern aus dem Cosmid M142 amplifiziert worden. Daraus entstanden die Konstrukte pBY456 (unc-119::α-Synuclein wt), pBY457 (unc-119::α-Synuclein A53T) und pBY458 (unc-119::α-Synuclein A30P).
Für die Herstellung der Konstrukte, die α-Synuclein unter der Kontrolle des sel-12-Promotors exprimieren, wurden die humane α-Synuclein-cDNA und die cDNAs der A53T und A30P Mutanten mit Hilfe der Restriktionsenzyme MscI und NcoI aus den Konstrukten pBY456, pBY457 bzw. pBY458 herausgeschnitten und in die MscI/ NcoI-Schnittstellen des Vektors pPD49.26 (Dr. Andrew Fire, Carnegie Institution of Washington, Baltimore, MD, USA) kloniert, in dem vorher der Promotor des C. elegans-Gens sel-12 eingebracht worden war. Die 5'-Promotor-Region vor dem sel-12 Gen war mittels PCR und den zwei Primern RB759 und RB110 (siehe unten) aus dem Cosmid C08A12 amplifiziert worden. Daraus entstanden die Konstrukte pBY1158 (sel-12::α-Synuclein wt), pBY1159 (sel-12::α-Synuclein A53T) und pBY1160 (sel-12::α-Synuclein A30P).

RB759: CCCGGCTGCAGCTCAATTATTCTAGTAAGC (SEQ ID NO:11)
RB110: GTCTCCATGGATCCGAATTCTGAAACGTTCAAATAAC (SEQ ID NO:12)

Der unc-119-Promotor führte zu einer panneuronalen Expression, während die Expression bei dem sel-12-Promotor neuronal und in einigen Muskelzellen erfolgte.

Herstellung transgener Tiere

Transgene wurden in C. elegans durch Mikroinjektion in die Gonaden (Mello et al., 1992) eingebracht. Die oben beschriebenen α-Synuclein-Konstrukte wurden mit den Markerplasmiden pBY1153 (sel-12::EGFP) bzw. pBY266 (dat::EGFP) coinjiziert und GFP-exprimierende Nachkommen selektioniert.
Stämme, die das Transgen stabil im Chromosom integriert besitzen, wurden durch Röntgenbestrahlung (5000 rad) von Linien gewonnen, die transgene, extrachromosomale Arrays enthielten. Die Nachkommen der röntgenbestrahlten Tiere wurden auf die 100%-ige Weitergabe des transgenen Markers durchsucht. Um eventuelle Hintergrundmutationen zu eliminieren, wurden die integrierten Linien mit Wildtypwürmern (N2) zurückgekreuzt.

PAGE und Immunoblotting

Die Extraktion der Proteine aus C. elegans, PAGE und Western Blotting wurden nach Okochi et al. (2000) durchgeführt. Für die Immunofärbung der Western Blots wurde der monoklonaler Antikörper 15G7 (Connex, Martinsried) in 1:4 Verdünnung verwendet. Als sekundärer Antikörper diente ein Peroxidase konjugierter Goat α Rat-Antikörper (Jackson) in der Verdünnung 1:5000.

Immunofärbungen

Die Immunofärbungen wurden nach einem Standardprotokoll (Finny et al. 1990) mit dem monoklonalen Antikörper 15G7 (Connex, Martinsried) unverdünnt durchgeführt. Als sekundärer Antikörper diente ein texas red gekoppelter Goat α Rat-Antikörper in der Verdünnung 1:2000.

α-Synuclein Aggregationen in Harnstoffextrakten in vitro

Harnstoffextrakte von Proteinen aus dem integrierten sel-12/α-Synuclein A30P-Wurm zeigen eine hochmolekulare Bande im Western Blot (Fig. 10) mit einem monoklonalen Antikörper gegen α-Synuclein, die in gleichbehandelten Extrakten aus N2-Würmern nicht sichtbar ist.

α-Synuclein Aggregationen in Neuronen in vivo

Die Immunofärbungen (Fig. 11) des unc-119/α-Synuclein A30P mit einem monoklonalen Antikörper gegen α-Synuclein zeigen deutliche Signale, sowohl im Zellkörper, wie auch in Neuriten, wobei dort oft perlenkettenartige Färbung zu sehen ist. Dies deutet auf den Transport des α-Synuclein Proteins zur Präsynapse und dessen Akkumulationen in den Axonen hin.

Defekte in der Eiablage und der Vulvaformation

Würmer, bei denen das Konstrukt sel-12/α-Synuclein A30P stabil im Genom integriert vorliegt, zeigen eine Vulvadeformation (p-vul) und einen Defekt in der Eiablage (egl) (Fig. 12).

Referenzliteratur:

1. Polymeropoulos, M.H. et al. Mutation in the alpha-synuclein gene identified in families with Parkinson's disease [see comments]. Science 276, 2045-7 (1997).
2. Kitada, T. et al. Mutations in the parkin gene cause autosomal recessive juvenile parkinsonism [see comments]. Nature 392, 605-8 (1998).
3. Baumeister, R., Liu, Y. & Ruvkun, G. Lineage-specific regulators couple cell lineage asymmetry to the transcription of the C.elegans POU gene unc-86 during neurogenesis. Genes Dev. 10, 1395-1410 (1996).
4. Way, J.C. & Chalfie, M. The mec-3 gene of Caenorhabditis elegans requires its own product for maintained expression and is expressed in three neuronal cell types. Genes Dev. 3, 1823-1833 (1989).
5. Hobert, O. et al. Regulation of interneuron function in the C. elegans thermoregulatory pathway by the ttx-3 LIM homeobox gene. Neuron 19, 345-57 (1997).
6. Dunnett, S.B. and Björklund, A. Prospects for new restorative and neuroprotective treatments in Parkinson's disease. Nature 399, A32-A39 (1999).
7. Hattori, N. et al. Molecular genetic analysis of a novel Parkin gene in Japanese families with autosomal recessive juvenile Parkinsonism: evidence for variable homozygous deletions in the Parkin gene in affected individuals. Ann. Neurol. 44, 935-941 (1998).
8. Liu, L.X. et al. High-throughput isolation of Caenorhabditis elegans deletion mutants. Genome Res. 9, 859-867 (1999).
9. Olanow, C.W. and Tatton, W.G. Etiology and pathogenesis of Parkinson's disease. Annu. Rev. Neurosci. 22, 123-144 (1999).
10. Yandell, M.D., Edgar, L.G. and Wood, W.B. Trimethylpsoralen induces small deletion mutations in Caenorhabditis elegans. Proc. Natl. Acad. Sci. 91, 1381-1385 (1994).
11. Clayton, D.F. and George, J.M. The synuclein: a family of proteins involved in synaptic function, plasticity, neurodegeneration and disease. TINS 21, 249-254 (1998).
12. Giasson, B.I., Murray, I., Trojanowski, J.Q. and Lee, V.M. A hydrophobic stretch of 12 amino acid residues in the middle of alpha-synuclein is essential for filament assembly. J. Biol. Chem. 26, 2380-2386 (2000).
13. Feany, M.B. and Bender, W.W. A parkinson model of parkinson's disease. Nature 404, 394-398 (2000).
14. Finney, M. and Ruvkun, G. The unc-86 gene product couples cell lineage and cell identity in C. elegans. Cell 63, 895-905 (1990).
15. Kruger, R. et al. Ala30Pro mutation in the gene encoding alpha-synuclein in Parkinson's disease. Nat Genet. 18, 106-8 (1998).
16. Masliah, E. et al. Dopaminergic loss and inclusion body formation in alpha-synuclein mice: implication for neurodegenerative disorders. Science 287, 1265-1269 (2000).
17. Mello, C.C., Kramer, J.M., Stinchcomb, D. and Ambros, V. Efficient gene transfer in C. elegans. Extrachromosomal maintainance and integration of transforming sequences. EMBO J. 10, 3959-3970 (1992).
18. Okochi, M. et al. A loss of function mutant of the presenilin homologue SEL-12 undergoes aberrant endoproteolysis in caenorhabditis elegans and increases abeta 42 generation in human cells. J. Biol. Chem. 275, 40925-32 (2000).
19. Zamore, P.D. et al., RNAi: Double-stranded RNA directs the ATP-dependent cleavage of mRNA at 21 to 23 nucleotide intervals. Cell 101, 25-33 (2000).

**Patentansprüche**

1. Nematode, der eine aberrante oder fehlende Expression mindestens eines Gens, das mit der Parkinsonschen Erkrankung in Zusammenhang steht, aufweist.

2. Nematode nach Anspruch 1, wobei das Gen ein Parkingen und/oder ein - Synucleingen ist.

3. Nematode nach Anspruch 2, dessen Parkingen ganz oder teilweise deletiert ist.

4. Nematode nach Anspruch 2, dessen Parkingen durch die RNA interference-Technik zumindest vorübergehend inaktiviert wurde.

5. Nematode nach Anspruch 2, in welchen ein humanes -Synucleingen eingeführt wurde.

6. Nematode nach einem der Ansprüche 1 bis 5, in dem ein humanes Parkingen oder ein dazu homologes Gen als Transgen exprimiert wird.

7. Nematode nach einem der Ansprüche 1 bis 5, in dem ein humanes - Synucleingen oder ein dazu homologes Gen als Transgen exprimiert wird.

8. Nematode nach einem der Ansprüche 1 bis 7, bei dem die aberrante Genexpression in mindestens einer der folgenden Formen erfolgt:

    - in Form einer verstärkten Genexpression, die zur Erzeugung einer gegenüber dem Wildtyp-Gen erhöhten Menge von Transkriptions- und/oder Translationsprodukt des betreffenden Gens führt,
    - in Form einer verringerten Genexpression, die zur Erzeugung einer gegenüber dem Wildtyp-Gen verringerten Menge von Transkriptions- und/oder Translationsprodukt des betreffenden Gens führt,
    - in Form einer Expression eines mutierten Gens oder
    - in Form einer Expression eines zu dem Organismus homologen oder heterologen Gens.

9. Nematode nach einem der Ansprüche 1 bis 8, der einen mit der aberranten oder fehlenden Genexpression des Parkin- und/oder -Synucleingens assoziierten Phänotyp aufweist.

10. Nematode nach Anspruch 9, wobei der Phänotyp, der mit der aberranten oder fehlenden Genexpression des Parkingens assoziiert ist, mindestens einer der folgenden ist:

    - Ein Chemotaxisdefekt,
    - Ein Eiablagedefekt,
    - Eine verlangsamte Entwicklungsdauer,
    - Eine verringerte Anzahl an Nachkommen,
    - Koordinationsprobleme,
    - Ein Defäkationsdefekt,

- Eine verlangsamte Fortbewegung, sowie
- Eine verkürzte Körperlänge.

**11.** Nematode nach Anspruch 9, wobei der Phänotyp, der mit der aberranten oder fehlenden Genexpression des -Synucleingens assoziiert ist, mindestens einer der folgenden ist:

- Ein Eiablagedefekt,
- Ein Defekt bei der Vulvaformation,
- Ablagerungen von -Synuclein,
- Eine verlangsamte Entwicklungsdauer, sowie
- Eine verringerte Anzahl an Nachkommen.

**12.** Nematode nach einem der Ansprüche 1 bis 11, der ein Nematode der Gattung Caenorhabditis, beispielsweise C. elegans, C. vulgaris oder C. briggsae, ist.

**13.** Verwendung des Nematoden nach einem der Ansprüche 1 bis 12 als Modellorganismus für neurodegenerative Erkrankungen.

**14.** Verwendung nach Anspruch 13, wobei die neurodegenerativen Erkrankungen derart sind, dass plaqueähnliche Ablagerungen im Nervensystem auftreten.

**15.** Verwendung nach Anspruch 13 oder 14, wobei die neurodegenerative Erkrankung die Parkinsonsche Erkrankung ist.

**16.** Verwendung des Organismus nach einem der Ansprüche 1 bis 12 zur Untersuchung der Mechanismen der Krankheitsentstehung, des Krankheitsverlaufs und/oder der Propagation von neurodegenerativen Erkrankungen, einschließlich jenen, bei denen plaqueähnliche Ablagerungen im Nervensystem auftreten, insbesondere der Parkinsonschen Erkrankung.

**17.** Verwendung des Organismus nach einem der Ansprüche 1 bis 12 zur Identifizierung und/oder Charakterisierung von Arzneimitteln zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung.

**18.** Verwendung des Organismus nach einem der Ansprüche 1 bis 12 zur Identifizierung und/oder Charakterisierung von Wirkstoffen, die in der Lage sind, die Wirkung von Arzneimitteln, die zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung eingesetzt werden, zu modifizieren.

**19.** Verfahren zur Untersuchung einer Substanz hinsichtlich Wirksamkeit bei der Behandlung und/oder Prävention von neurodegenerativen Erkrankungen, einschließlich jenen, bei denen plaqueähnliche Ablagerungen im Nervensystem auftreten, welches umfasst, einen Nematoden nach einem der Ansprüche 1 bis 12 der Substanz auszusetzen und Veränderungen des Phänotyps zu bestimmen.

**20.** Verfahren zur Untersuchung einer Substanz hinsichtlich einer Eignung, die Wirkung eines Arzneimittels bei der Behandlung und/oder Prävention von neurodegenerativen Erkrankungen, einschließlich jenen, bei denen plaqueähnliche Ablagerungen im Nervensystem auftreten, zu modifizieren, wobei das Verfahren umfasst,

- einen Nematoden nach einem der Ansprüche 1 bis 12 dem Arzneimittel auszusetzen und die aufgrund der Arzneimittelwirkung auftretenden Veränderungen des Phänotyps zu bestimmen,
- mindestens ein weiteres Exemplar des Nematoden der in dem vorigen Schritt verwendeten Art dem Arzneimittel in Gegenwart der Substanz auszusetzen und gegebenenfalls auftretenden Veränderungen des Phänotyps mit den aufgrund der Arzneimittelwirkung allein auftretenden Veränderungen des Phänotyps zu vergleichen.

**21.** Substanz mit Wirksamkeit bei der Behandlung und/oder Prävention von neurodegenerativen Erkrankungen, einschließlich jenen, bei denen plaqueähnliche Ablagerungen im Nervensystem auftreten, oder Substanz mit einer Eignung, die Wirkung eines Arzneimittels bei der Behandlung und/oder Prävention von neurodegenerativen Erkrankungen, einschließlich jenen, bei denen plaqueähnliche Ablagerungen im Nervensystem auftreten, zu modifizieren, die mittels eines Verfahrens nach einem der Ansprüche 19 oder 20 identifiziert worden ist.

**22.** Verwendung des Nematoden nach einem der Ansprüche 1 bis 12 zur Identifizierung und/oder Charakterisierung von weiteren Genen, die in Zusammenhang mit den Mechanismen der Krankheitsentstehung, des Krankheitsverlaufs und/oder der Propagation von neurodegenerativen Erkrankungen, einschließlich jenen, bei denen plaqueähnliche Ablagerungen im Nervensystem auftreten, insbesondere der Parkinsonschen Erkrankung, stehen.

**23.** Verwendung des Nematoden nach einem der Ansprüche 1 bis 12 zur Identifizierung und/oder Charakterisierung von Genen, die in mutiertem oder unmutiertem Zustand zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung wirksam sind.

**24.** Verwendung des Nematoden nach einem der Ansprüche 1 bis 12 zur Identifizierung und/oder Charakterisierung von Genen, die in mutiertem oder unmutiertem Zustand in der Lage sind, die Wirkung von Arzneimitteln und/oder Genen, die zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung eingesetzt werden, zu modifizieren.

**25.** Verfahren zur Identifizierung und/oder Charakterisierung von Genen, die in mutiertem oder unmutiertem Zustand zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung wirksam sind, welches die folgenden Schritte umfasst,

- an Nematoden nach einem der Ansprüche 1 bis 12 eine ungerichtete Mutagenese vorzunehmen,
- an den aus der Mutagenese resultierenden Nematoden gegebenenfalls feststellbare Veränderungen des Phänotyps, der mit der aberranten oder fehlenden Genexpression des mindestens einen mit derartigen Erkrankungen in Verbindung stehenden Gens assoziiert ist, verglichen mit den für die ungerichtete Mutagenese verwendeten Ausgangsnematoden zu bestimmen und
- das oder die Gene, die infolge ihrer Mutation für die in dem vorangegangenen Schritt beobachteten Veränderungen des Phänotyps verantwortlich sind, zu identifizieren.

**26.** Verfahren zur Identifizierung und/oder Charakterisierung von Genen, die in mutiertem oder unmutiertem Zustand in der Lage sind, die Wirkung von Arzneimitteln und/oder Genen, die zur Prävention oder Behandlung von neurodegenerativen Erkrankungen, einschließlich jenen, bei denen plaqueähnliche Ablagerungen im Nervensystem auftreten, insbesondere der Parkinsonschen Erkrankung eingesetzt werden, zu modifizieren, welches die Schritte umfasst:

- an Nematoden nach einem der Ansprüche 1 bis 12, eine ungerichtete Mutagenese vorzunehmen,
- aus den aus der Mutagenese resultierenden Nematoden Nachkommen identischer Art zu erzeugen,
- an den jeweils identischen Nachkommen einen gegebenenfalls vorliegenden Phänotyp, der mit der aberranten oder fehlenden Genexpression des mindestens einen mit derartigen Erkrankungen in Verbindung stehenden Gens assoziiert ist, zu bestimmen,
- den in dem vorangegangenen Schritt an den Nachkommen bestimmten Phänotyp mit dem entsprechenden Phänotyp der Ausgangsnematoden nach einem der Ansprüche 1 bis 12 zu vergleichen,
- an den jeweils identischen Nachkommen, die den gleichen Phänotyp wie die Ausgangsnematoden zeigen, Veränderungen des Phänotyps, der mit der aberranten oder fehlenden Genexpression des mindestens einen mit derartigen Erkrankungen in Verbindung stehenden Gens assoziiert ist, in Gegenwart des zu untersuchenden Arzneimittels oder bei Expression des zu untersuchenden Gens zu bestimmen, und
- die bestimmten Veränderungen des Phänotyps mit den Veränderungen des Phänotyps der Ausgangsnematoden in Gegenwart des zu untersuchenden Arzneimittels oder bei Expression des zu untersuchenden Gens zu vergleichen, um gegebenenfalls auftretende Abweichungen der Phänotypveränderung festzustellen,
- das oder die Gene, die infolge ihrer Mutation für die in dem vorangegangenen Schritt gegebenenfalls festgestellten Abweichungen der Phänotypveränderungen verantwortlich sind, zu identifizieren.

**27.** Gen mit Wirksamkeit in mutiertem oder unmutiertem Zustand bei der Behandlung und/oder Prävention von neurodegenerativen Erkrankungen, einschließlich jenen, bei denen plaqueähnliche Ablagerungen im Nervensystem auftreten, das mittels eines Verfahrens nach einem der Ansprüche 25 oder 26 identifiziert worden ist.

**28.** Isoliertes Nukleinsäuremolekül, das Parkin aus Caenorhabditis elegans mit der in Fig. 3 angegebenen Aminosäuresequenz kodiert.

**29.** Isoliertes Nukleinsäuremolekül nach Anspruch 28, das die in Fig. 3 angegebene Nukleinsäuresequenz oder eine dazu komplementäre Nukleinsäuresequenz aufweist.

**30.** Isoliertes Nukleinsäuremolekül, dessen Nukleotidsequenz mindestens 75% Sequenzähnlichkeit zu dem isolierten Nukleinsäuremolekül nach Anspruch 29 aufweist.

**31.** Isoliertes Nukleinsäuremolekül, das unter stringenten Bedingungen mit dem isolierten DNA-Molekül nach Anspruch 29 hybridisiert.

**32.** Fragment eines isolierten Nukleinsäuremoleküls nach einem der Ansprüche 28 bis 31.

**33.** Vektor, Plasmid, Cosmid, Bacmid, YAC, BAC, Virus- oder Phagengenom, der bzw. das ein isoliertes Nukleinsäuremolekül oder ein Fragment nach einem der Ansprüche 28 bis 32 enthält.

**34.** Polypeptid oder Protein, das von einem Nukleinsäuremolekül oder einem Fragment nach einem der Ansprüche 28-32 kodiert wird.

**35.** Transgener Organismus, insbesondere Mikroorganismus, Pflanze oder Tier, oder Teil oder Vermehrungsmaterial eines transgenen Organismus, der bzw. das eine rekombinante Nukleinsäuresequenz umfasst, die ein Nukleinsäuremolekül oder ein Fragment nach einem der Ansprüche 28 bis 32 als Transgen enthält.

**FIG. 1**

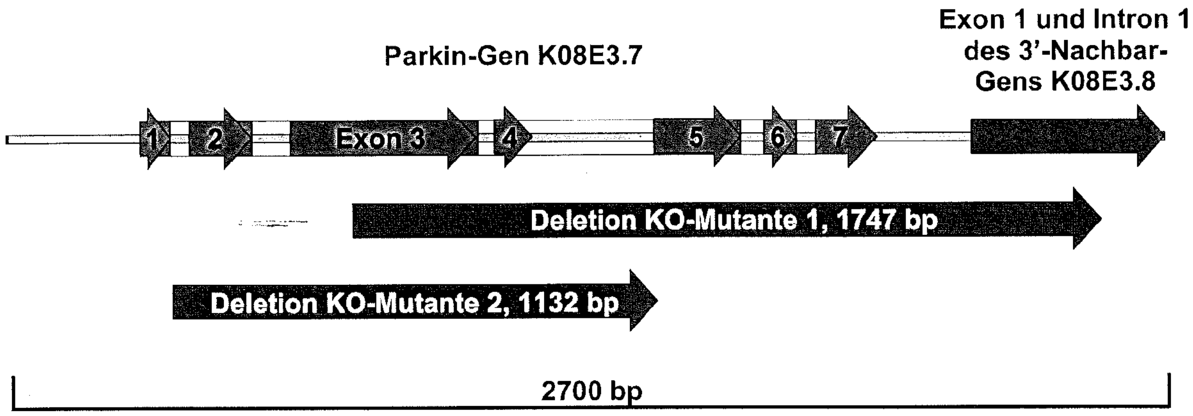
Exon 1 und Intron 1
des 3'-Nachbar-
Gens K08E3.8
Parkin-Gen K08E3.7
1
2
Exon 3
4
5
6
7
Deletion KO-Mutante 1, 1747 bp
Deletion KO-Mutante 2, 1132 bp
2700 bp

**FIG. 2**

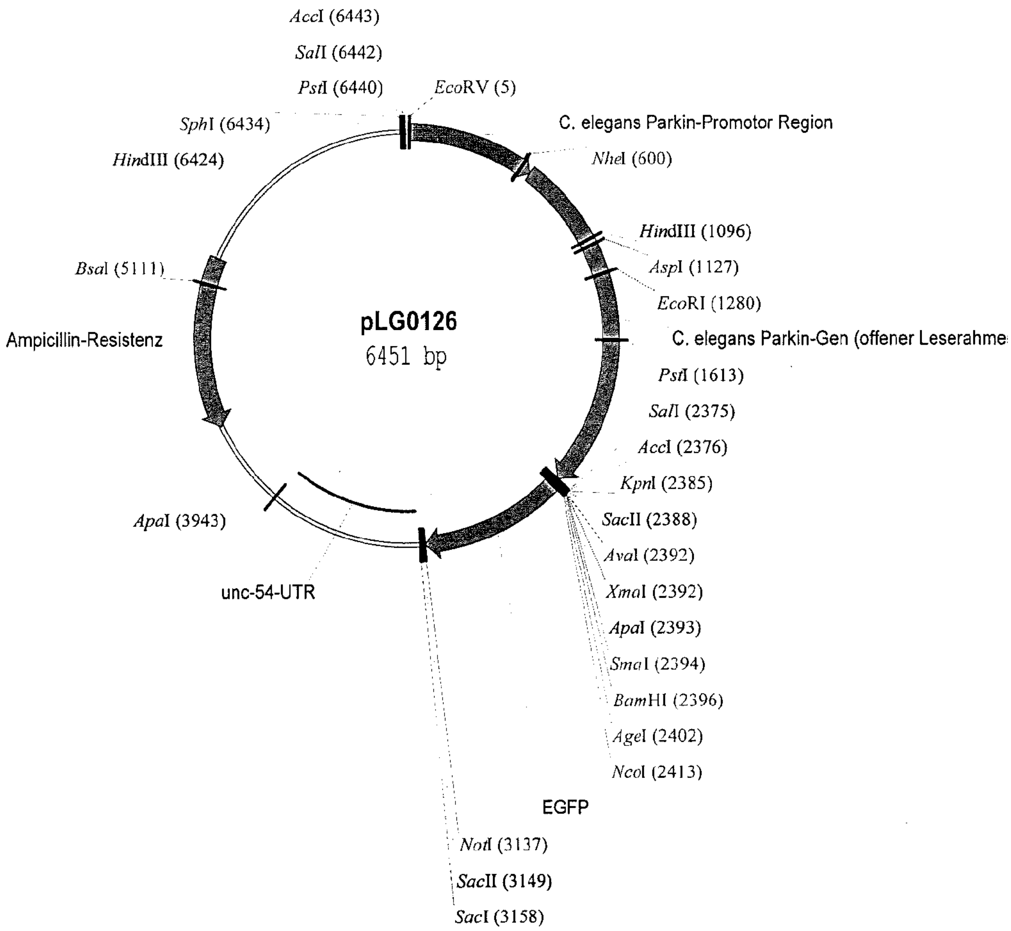
AccI (6443)
SalI (6442)
PstI (6440)
EcoRV (5)
SphI (6434)
C. elegans Parkin-Promotor Region
HindIII (6424)
NheI (600)
HindIII (1096)
AspI (1127)
BsaI (5111)
EcoRI (1280)
Ampicillin-Resistenz
pLG0126
6451 bp
C. elegans Parkin-Gen (offener Leserahme
PstI (1613)
SalI (2375)
AccI (2376)
KpnI (2385)
ApaI (3943)
SacII (2388)
AvaI (2392)
unc-54-UTR
XmaI (2392)
ApaI (2393)
SmaI (2394)
BamHI (2396)
AgeI (2402)
NcoI (2413)
EGFP
NotI (3137)
SacII (3149)
SacI (3158)

# FIG. 3

Parkin-cDNA von *C. elegans*, kodierender Bereich.

atgtctgatgaaatctctatattaatacaagatagaaaaacaggtcaacgtaggaatctaacacttaatataaata
taactggaaatatcgaagatctcacaaaagatgtggaaaagctcaccgaaattcccagcgatgagctggaagtggt
tttctgtgggaaaaagttatcaaaatcaacgattatgaggatttgtcactgacacctgcaacacaaatcatgctt
ctccgtccaaagttcaatagtcacaacgaaaacggtgctactactgcaaaaataacaacagattcttcaattctcg
gaagcttctacgtgtggtgcaaaaattgtgacgacgtcaagcgcggcaaactgcgggtttattgccaaaaatgctc
gtcaacctctgttctagtcaaatctgaaccccagaactggtccgacgttctcaaaagcaagagaataccggcggtc
tgcgaagaatgctgtactccaggtcttttcgctgaattcaagttcaaatgtctagcctgcaacgatccggccgcag
ctctaactcacgtacgcggaaattggcaaatgaccgagtgctgtgtttgtgatgggaaggagaaagtgatcttcga
cctcggatgcaatcatattacatgccaattctgtttcagaGATTATTTGCTAAGTCAACTGGAACGATTCGGTTTT
GTCAATCAGCCGCCGCATGGCTTCACCATTTTCTGCCCCTATCCAGGGTGCAATAgagtggtacaagatgtgcacc
atttccacattatgggtcagacgtcgtacagcgaataccaacggaaagccaccgagcgattgattgccgtggacga
caagggtgtgacttgcccgaatgtctcgtgtgggcagagcttcttctgggagccctatgatgacgatggaagatcc
cagtgtccagattgtttttttttcgttttgcagaaagtgcttcgaaagaaattgtgtgtgccagagcgaagacgatc
tcacccgaactacaattgacgcgactacaagaagatgcccaaaatgccacgtggcaaccgaacggaacggcggatg
tgctcacattcactgtacctcgtgtggaatggattggtgtttcaagtgcaagacagaatggaaggaagagtgtcaa
tgggaccattggtttaattaa

Abgeleitete Aminosäuresequenz von *C.elegans*-Parkin

MSDEISILIQDRKTGQRRNLTLNINITGNIEDLTKDVEKLTEIPSDELEVVFCGKKLSKSTIMRDLSLTPATQIML
LRPKFNSHNENGATTAKITTDSSILGSFYVWCKNCDDVKRGKLRVYCQKCSSTSVLVKSEPQNWSDVLKSKRIPAV
CEECCTPGLFAEFKFKCLACNDPAAALTHVRGNWQMTECCVCDGKEKVIFDLGCNHITCQFCFRDYLLSQLERFGF
VNQPPHGFTIFCPYPGCNRVVQDVHHFHIMGQTSYSEYQRKATERLIAVDDKGVTCPNVSCGQSFFWEPYDDDGRS
QCPDCFFSFCRKCFERNCVCQSEDDLTRTTIDATTRRCPKCHVATERNGGCAHIHCTSCGMDWCFKCKTEWKEECQ
WDHWFN

**FIG. 4a**: Expression von GFP unter der Kontrolle von 4085 bp des Parkin-Promotors.

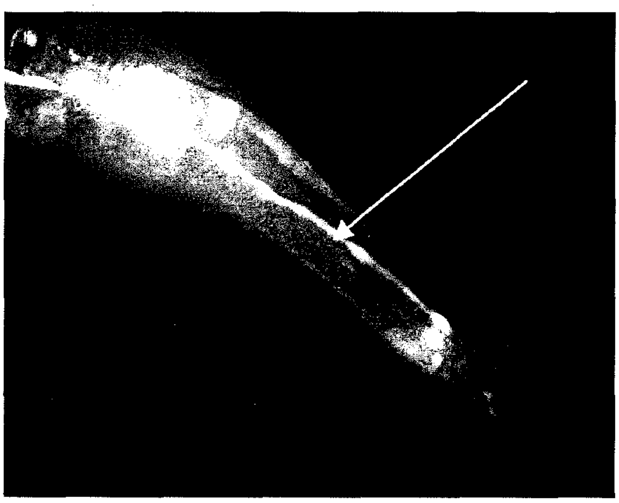

Expression in Neuronen des posterioren Bereichs von C.elegans. Pfeil: ventral nerve cord Neuriten .

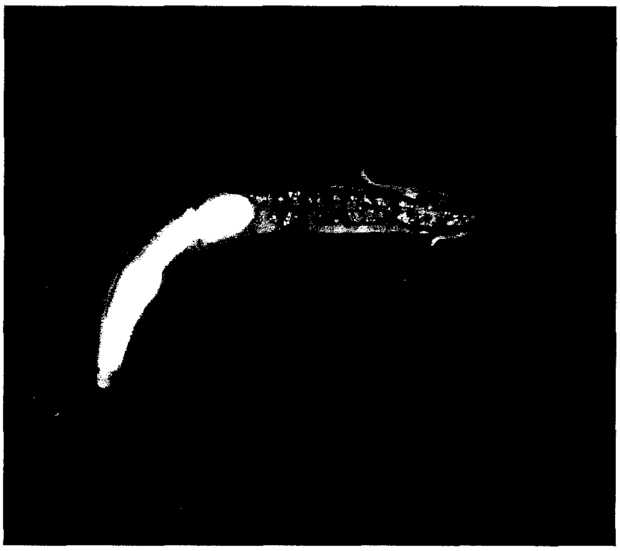

Expression in allen Zellen des Pharynx

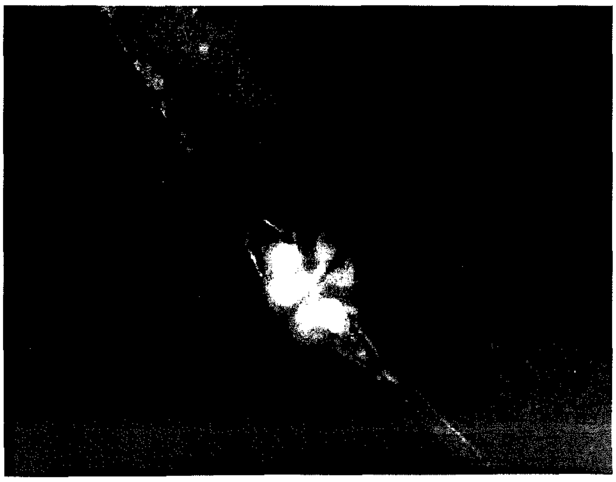

Expression in allen Zellen der Vulva.

# Figur 4b

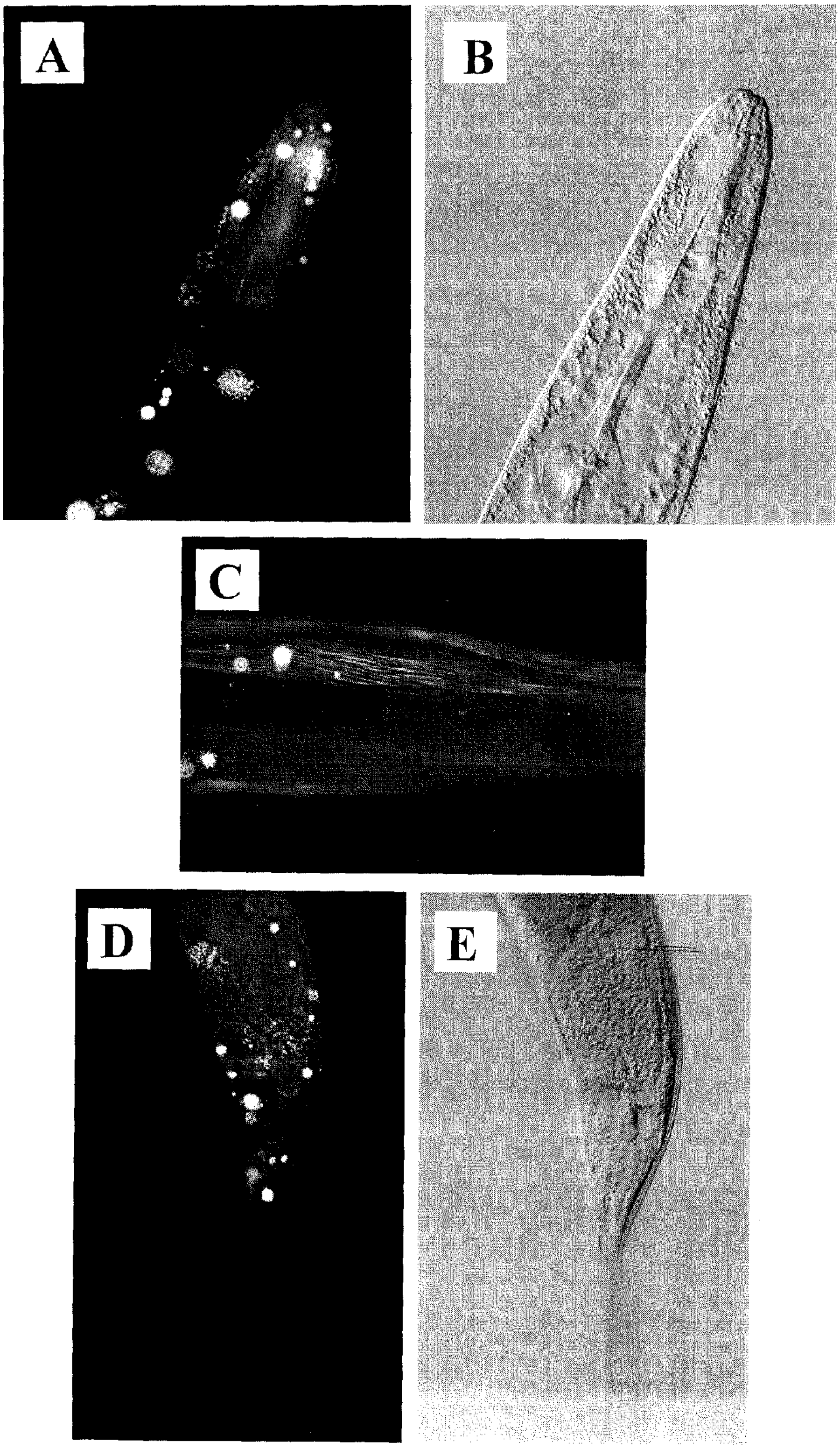

**FIG. 5**

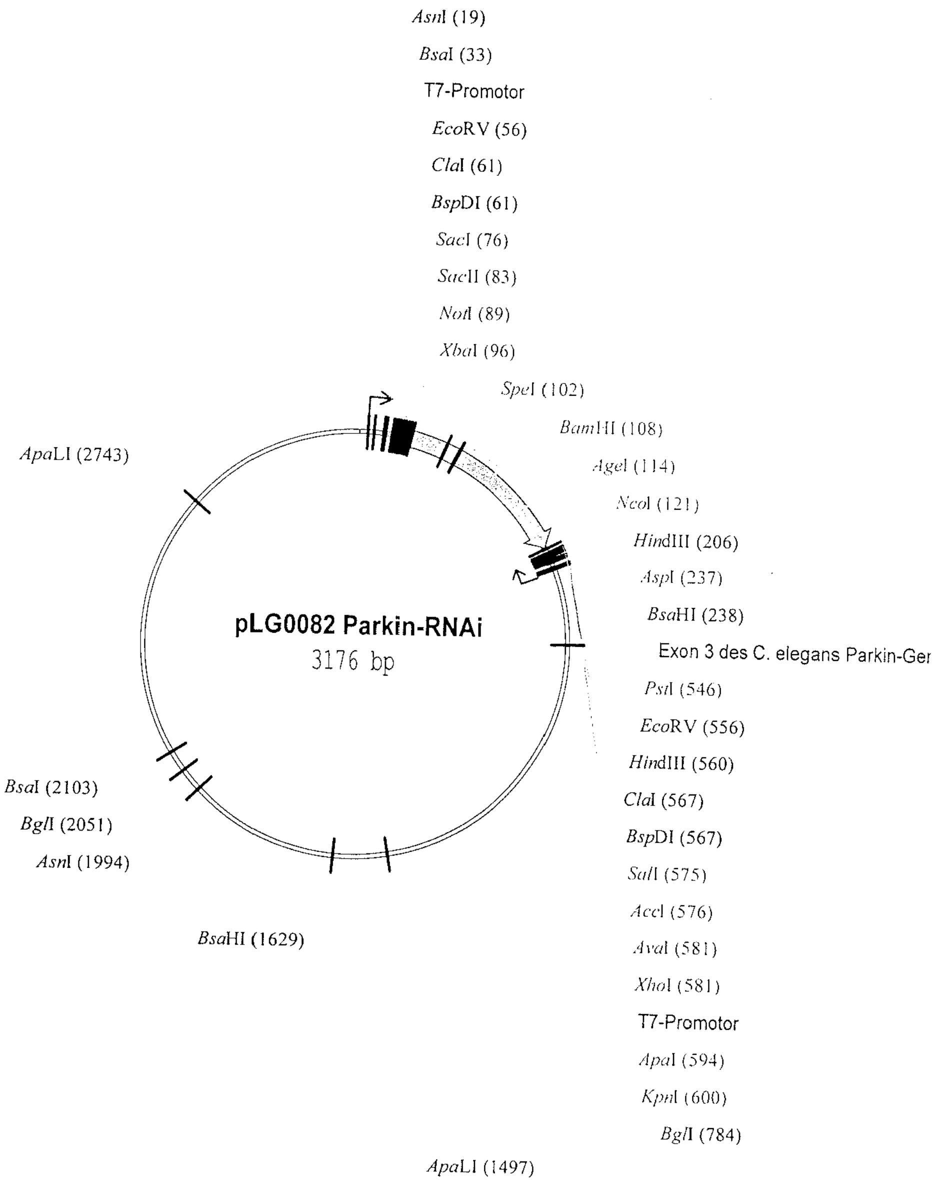
AsnI (19)
BsaI (33)
T7-Promotor
EcoRV (56)
ClaI (61)
BspDI (61)
SacI (76)
SacII (83)
NotI (89)
XbaI (96)
SpeI (102)
BamHI (108)
AgeI (114)
NcoI (121)
HindIII (206)
AspI (237)
BsaHI (238)
Exon 3 des C. elegans Parkin-Ger
PstI (546)
EcoRV (556)
HindIII (560)
ClaI (567)
BspDI (567)
SalI (575)
AccI (576)
AvaI (581)
XhoI (581)
T7-Promotor
ApaI (594)
KpnI (600)
BglI (784)
ApaLI (1497)
BsaHI (1629)
AsnI (1994)
BglI (2051)
BsaI (2103)
ApaLI (2743)
pLG0082 Parkin-RNAi
3176 bp

FIG. 6

a)

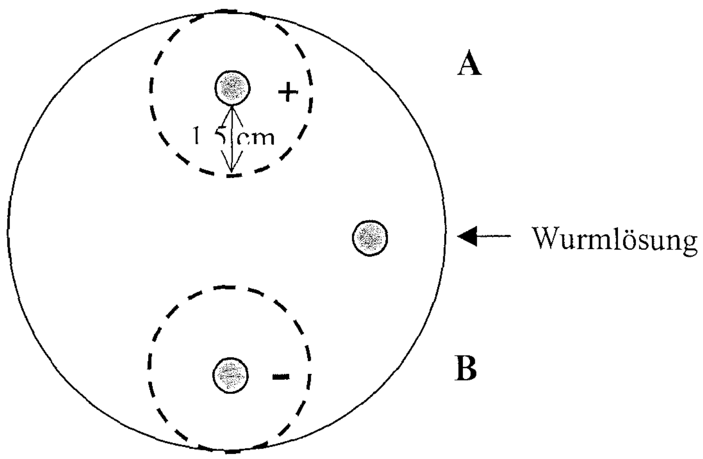


b)

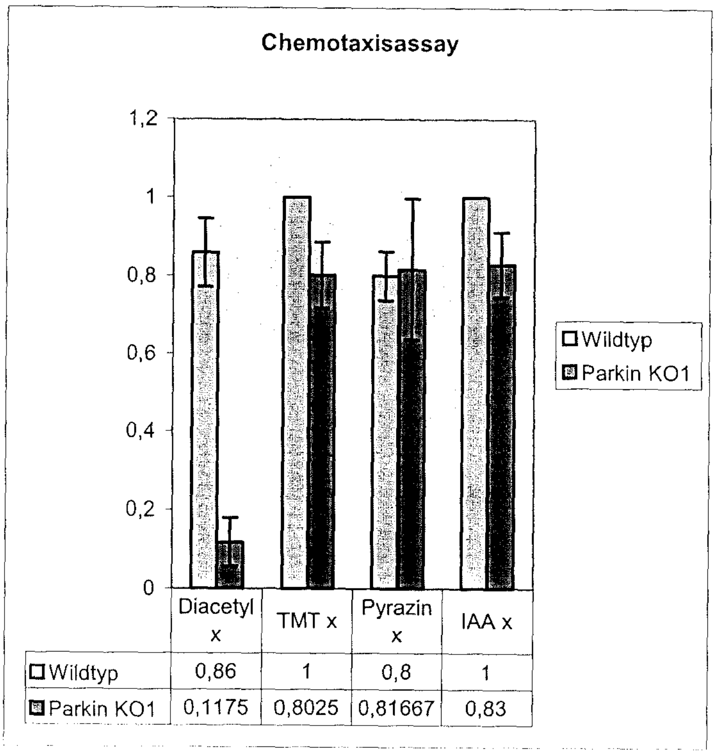


**Diacetyl, TMT Pyrazin, IAA statistisch relevant (Student's t-test) p<0.0001**

**FIG. 7**

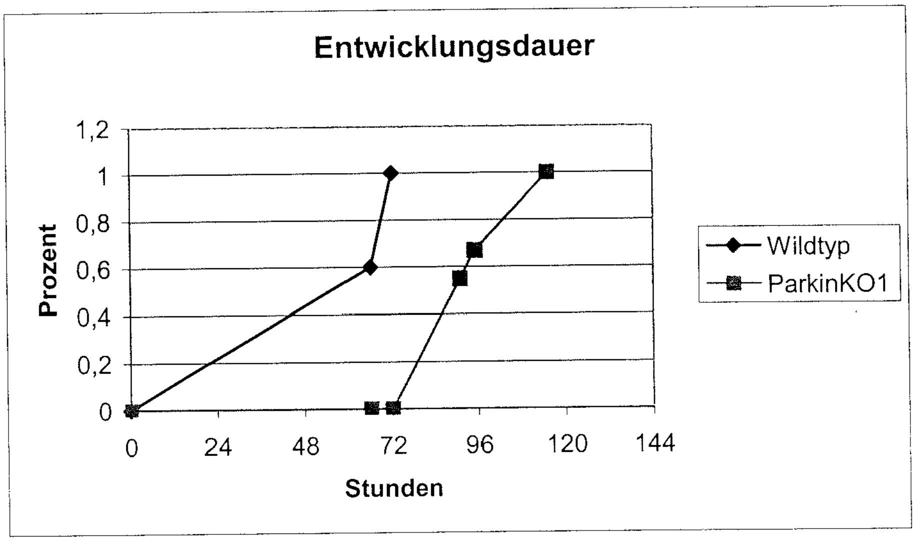


**FIG. 8**

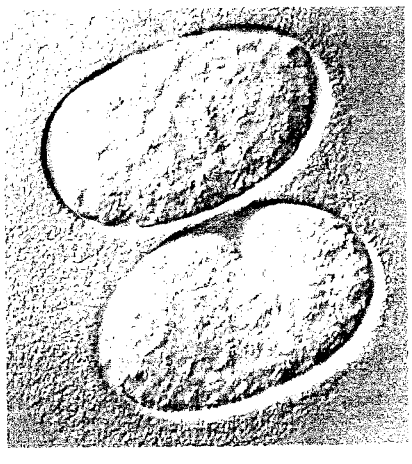

Normales Stadium, in dem Eier gelegt werden (Gastrulation)

Morphogenese (Bohnenstadium)

**FIG. 9**

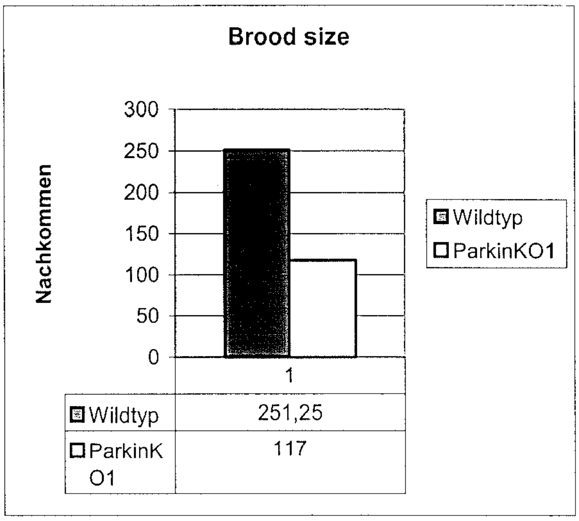
Brood size
Nachkommen
300
250
200
150
100
50
0
1
Wildtyp
ParkinKO1
Wildtyp
251,25
ParkinK O1
117

Figur 10

# Hochmolekulargewichts-Aggregate von α- Synuclein in *C. elegans*

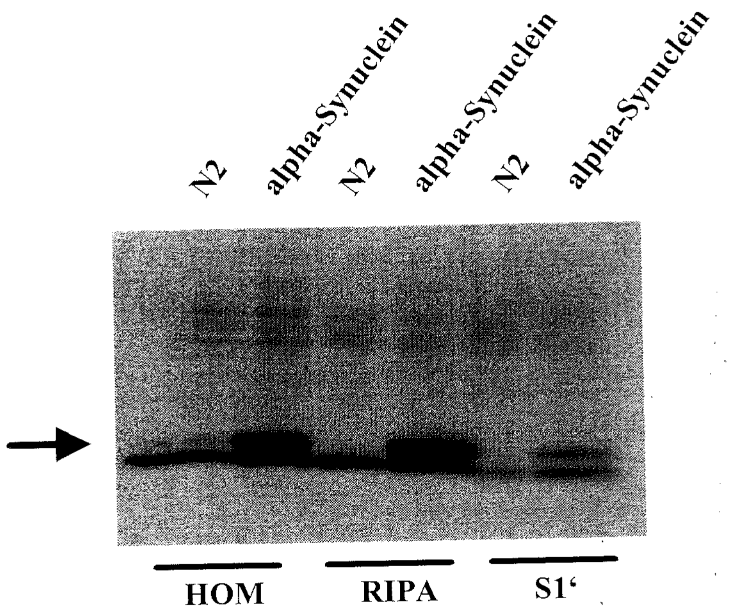


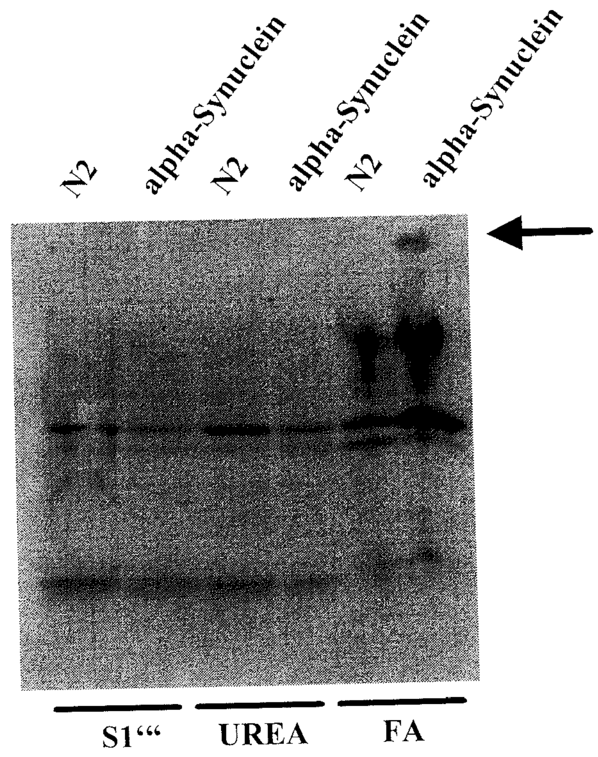

α-Synuclein Immunoreaktivität in Neuronen

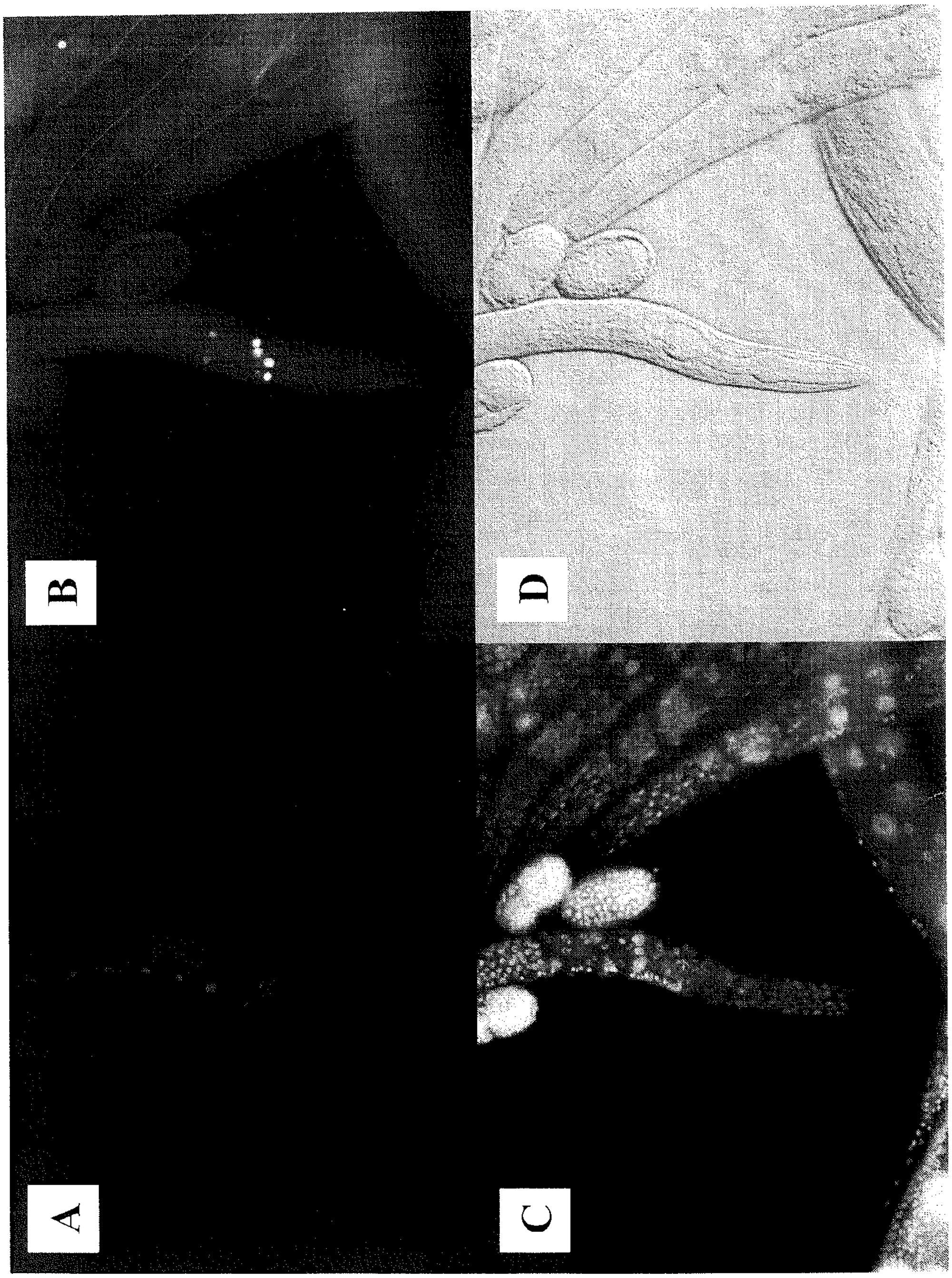


Figur 11

Figur 12

**Expression von α- Synuclein in *C. elegans* verursacht Eierablagedefekt und Vulve-Deformation**

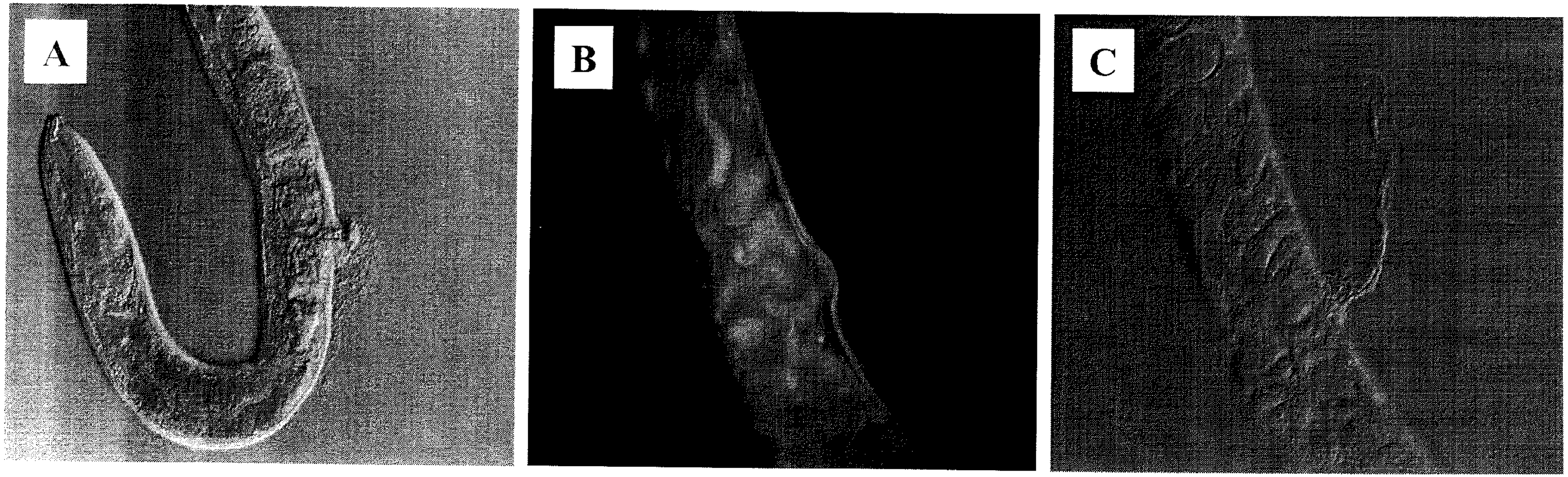

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **POLYMEROPOULOS, M.H. et al.** Mutation in the alpha-synuclein gene identified in families with Parkinson's disease [see comments. *Science,* 1997, vol. 276, 2045-7
- **KITADA, T. et al.** Mutations in the parkin gene cause autosomal recessive juvenile parkinsonism [see comments. *Nature,* 1998, vol. 392, 605-8
- **BAUMEISTER, R. ; LIU, Y. ; RUVKUN, G.** Lineage-specific regulators couple cell lineage asymmetry to the transcription of the C.elegans POU gene unc-86 during neurogenesis. *Genes Dev.,* 1996, vol. 10, 1395-1410
- **WAY, J.C. ; CHALFIE, M.** The mec-3 gene of Caenorhabditis elegans requires its own product for maintained expression and is expressed in three neuronal cell types. *Genes Dev.,* 1989, vol. 3, 1823-1833
- **HOBERT, O. et al.** Regulation of interneuron function in the C. elegans thermoregulatory pathway by the ttx-3 LIM homeobox gene. *Neuron,* 1997, vol. 19, 345-57
- **DUNNETT, S.B. ; BJÖRKLUND, A.** Prospects for new restorative and neuroprotective treatments in Parkinson's disease. *Nature,* 1999, vol. 399, A32-A39
- **HATTORI, N. et al.** Molecular genetic analysis of a novel Parkin gene in Japanese families with autosomal recessive juvenile Parkinsonism: evidence for variable homozygous deletions in the Parkin gene in affected individuals. *Ann. Neurol.,* 1998, vol. 44, 935-941
- **LIU, L.X. et al.** High-throughput isolation of Caenorhabditis elegans deletion mutants. *Genome Res.,* 1999, vol. 9, 859-867
- **OLANOW, C.W. ; TATTON, W.G.** Etiology and pathogenesis of Parkinson's disease. *Annu. Rev. Neurosci.,* 1999, vol. 22, 123-144
- **YANDELL, M.D. ; EDGAR, L.G. ; WOOD, W.B.** Trimethylpsoralen induces small deletion mutations in Caenorhabditis elegans. *Proc. Natl. Acad. Sci.,* 1994, vol. 91, 1381-1385
- **CLAYTON, D.F. ; GEORGE, J.M.** The synuclein: a family of proteins involved in synaptic function, plasticity, neurodegeneration and disease. *TINS,* 1998, vol. 21, 249-254
- **GIASSON, B.I. ; MURRAY, I. ; TROJANOWSKI, J.Q. ; LEE, V.M.** A hydrophobic stretch of 12 amino acid residues in the middle of alpha-synuclein is essential for filament assembly. *J. Biol. Chem.,* 2000, vol. 26, 2380-2386
- **FEANY, M.B. ; BENDER, W.W.** A parkinson model of parkinson's disease. *Nature,* 2000, vol. 404, 394-398
- **FINNEY, M. ; RUVKUN, G.** The unc-86 gene product couples cell lineage and cell identity in C. elegans. *Cell,* 1990, vol. 63, 895-905
- **KRUGER, R. et al.** Ala30Pro mutation in the gene encoding alpha-synuclein in Parkinson's disease. *Nat Genet.,* 1998, vol. 18, 106-8
- **MASLIAH, E. et al.** Dopaminergic loss and inclusion body formation in alpha-synuclein mice: implication for neurodegenerative disorders. *Science,* 2000, vol. 287, 1265-1269
- **MELLO, C.C. ; KRAMER, J.M. ; STINCHCOMB, D. ; AMBROS, V.** Efficient gene transfer in C. elegans. Extrachromosomal maintainance and integration of transforming sequences. *EMBO J.,* 1992, vol. 10, 3959-3970
- **OKOCHI, M. et al.** A loss of function mutant of the presenilin homologue SEL-12 undergoes aberrant endoproteolysis in caenorhabditis elegans and increases abeta 42 generation in human cells. *J. Biol. Chem.,* 2000, vol. 275, 40925-32
- **ZAMORE, P.D. et al.** RNAi: Double-stranded RNA directs the ATP-dependent cleavage of mRNA at 21 to 23 nucleotide intervals. *Cell,* 2000, vol. 101, 25-33